(19) Europäisches Patentamt
European Patent Office
Office européen des brevets

(11) EP 1 385 825 B1

(12) # EUROPÄISCHE PATENTSCHRIFT

(45) Veröffentlichungstag und Bekanntmachung des Hinweises auf die Patenterteilung:
**15.08.2007 Patentblatt 2007/33**

(21) Anmeldenummer: **02750900.9**

(22) Anmeldetag: **08.05.2002**

(51) Int Cl.:
***C07D 213/36*** *(2006.01)* ***A61K 31/435*** *(2006.01)*
***A61P 25/00*** *(2006.01)*

(86) Internationale Anmeldenummer:
**PCT/EP2002/005078**

(87) Internationale Veröffentlichungsnummer:
**WO 2002/090330 (14.11.2002 Gazette 2002/46)**

(54) **SUBSTITUIERTE 2-PYRIDIN-CYCLOHEXAN-1,4-DIAMINDERIVATE**

SUBSTITUTED 2-PYRIDINE-CYCLOHEXANE-1,4-DIAMINE DERIVATIVES

DERIVES SUBSTITUES DE 2-PYRIDINE-CYCLOHEXANE-1,4-DIAMINE

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **09.05.2001 DE 10123163**

(43) Veröffentlichungstag der Anmeldung:
**04.02.2004 Patentblatt 2004/06**

(73) Patentinhaber: **Grünenthal GmbH**
**52078 Aachen (DE)**

(72) Erfinder:
- **SUNDERMANN, Bernd**
**52066 Aachen (DE)**
- **MAUL, Corinna**
**52066 Aachen (DE)**
- **BUSCHMANN, Helmut**
**E-08950 Esplugues de Llobregat (ES)**
- **HELLER, Barbara**
**18334 Dettmannsdorf (DE)**

(56) Entgegenhaltungen:
**WO-A-99/36421** **US-A- 4 113 866**

- **BERTORELLI R ET AL: "Nociceptin/orphanin FQ and its receptor: a potential target for drug discovery" TRENDS IN PHARMACOLOGICAL SCIENCES, ELSEVIER TRENDS JOURNAL, CAMBRIDGE, GB, Bd. 21, Nr. 7, 1. Juli 2000 (2000-07-01), Seiten 233-234, XP004209778 ISSN: 0165-6147**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 385 825 B1

Printed by Jouve, 75001 PARIS (FR)

## Beschreibung

Die vorliegende Erfindung betrifft substituierte 2-Pyridin-Cyclohexan-1,4-diaminderivate, Verfahren zu deren Herstellung, Arzneimittel enthaltend diese Verbindungen und die Verwendung von substituierten 2-Pyridin-Cyclohexan-1,4-diaminderivaten zur Herstellung von Arzneimitteln.

Das Heptadekapeptid Nociceptin ist ein endogener Ligand des ORL1 (Opioid-Receptor-Like)-Rezeptors (Meunier et al., Nature 377, 1995, S. 532-535), der zu der Familie der Opioid Rezeptoren gehört und in vielen Regionen des Gehirns und des Rückenmarks zu finden ist (Mollereau et al., FEBS Letters, 341, 1994, S. 33-38, Darland et al., Trends in Neurosciences, 21, 1998, S. 215-221). Das Peptid ist durch eine hohe Affinität, mit einem $K_d$-Wert von annähernd 56 pM (Ardati et al., Mol. Pharmacol. 51, S. 816-824), und durch eine hohe Selektivität für den ORL1-Rezeptor gekennzeichnet. Der ORL1-Rezeptor ist homolog zu den $\mu$, $\kappa$ und $\delta$ Opioid-Rezeptoren und die Aminosäuresequenz des Nociceptin-Peptids weist eine starke Ähnlichkeit mit denen der bekannten Opioidpeptide auf. Die durch das Nociceptin induzierte Aktivierung des Rezeptors führt über die Kopplung mit $G_{i/o}$-Proteinen zu einer Inhibierung der Adenylatcyclase (Meunier et al., Nature 377, 1995, S. 532-535). Auch auf der zellulären Ebene sind funktionelle Ähnlichkeiten der $\mu$, $\kappa$ und $\delta$ Opioid-Rezeptoren mit dem ORL1-Rezeptor in Bezug auf die Aktivierung des Kalium-Kanals (Matthes et al., Mol. Pharmacol. 50, 1996, S. 447-450; Vaughan et al., Br. J. Pharmacol. 117, 1996, S. 1609-1611) und der Inhibierung der L-, N- und P/Q-Typ-Kalzium-Kanäle vorhanden (Conner et al., Br. J. Pharmacol. 118, 1996, S. 205-207; Knoflach et al., J. Neuroscience 16, 1996, S. 6657-6664).

Das Nociceptin-Peptid zeigt nach intercerebroventicularer Applikation eine pronociceptive und hyperalgetische Aktivität in verschiedenen Tiermodellen (Reinscheid et al., Science 270, 1995, S. 792-794; Hara et al,. Br. J. Pharmacol. 121, 1997, S. 401-408). Diese Befunde können als Hemmung der stressinduzierten Analgesie erklärt werden (Mogil et al., Neurosci. Letters 214, 1996, S131-134; sowie Neuroscience 75, 1996, S. 333-337). In diesem Zusammenhang konnte auch eine anxiolytische Aktivität des Nociceptin nachgewiesen werden (Jenck et al., Proc. Natl. Acad. Sci. USA 94, 1997, 14854-14858).

Auf der anderen Seite konnte in verschiedenen Tiermodellen, insbesondere nach intrathekaler Applikation, auch ein antinociceptiver Effekt von Nociceptin gezeigt werden. Nociceptin hemmt die Aktivität Kainat- oder Glutamat-stimulierter Hinterwurzeiganglienneuronen (Shu et al., Neuropeptides, 32, 1998, 567-571) oder Glutamat-stimulierter Rückenmarksneuronen (Faber et al., Br. J. Pharmacol., 119, 1996, S. 189-190); es wirkt antinociceptiv im Tail Flick-Test in der Maus (King et al., Neurosci. Lett., 223, 1997, 113-116), im Flexor-Reflex-Modell in der Ratte (Xu et al., NeuroReport, 7, 1996, 2092-2094) und im Formalin-Test an der Ratte (Yamamoto et al., Neuroscience, 81, 1997, S. 249-254). In Modellen für neuropathische Schmerzen konnte ebenfalls eine antinociceptive Wirkung von Nociceptin nachgewiesen werden (Yamamoto und Nozaki-Taguchi, Anesthesiology, 87,1997), die insofern besonders interessant ist, als das die Wirksamkeit von Nociceptin nach Axotomie von Spinalnerven zunimmt. Dies steht im Gegensatz zu den klassischen Opioiden, deren Wirksamkeit unter diesen Bedingungen abnimmt (Abdulla und Smith, J. Neurosci., 18,1998, S. 9685-9694).

Der ORL1-Rezeptor ist außerdem noch an der Regulation weiterer physiologischer und pathophysiologischer Prozesse beteiligt. Hierzu gehören unter anderem Lernen und Gedächtnisbildung (Sandin et al., Eur. J. Neurosci., 9, 1997, S. 194-197; Manabe et al., Nature, 394, 1997, S. 577-581), Hörvermögen (Nishi et al., EMBO J., 16, 1997, S. 1858-1864), Nahrungsaufnahme (Pomonis et al., NeuroReport, 8, 1996, S. 369-371), Regulation des Blutdruckes (Gumusel et al., Life Sci., 60, 1997, S. 141-145; Campion und Kadowitz, Biochem. Biophys. Res. Comm., 234, 1997, S. 309-312), Epilepsie (Gutiérrez et al, Abstract 536.18, Society for Neuroscience, Vol 24, 28th Ann. Meeting, Los Angeles, November 7.-12, 1998) und Diurese (Kapista et al., Life Sciences, 60, 1997, PL 15-21). In einem Übersichtsartikel von Calo et al. (Br.J. Pharmacol., 129, 2000, 1261 -1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.:Analgesie, Stimulation und Regulation der Nahrungsaufnahme, Einfluß auf $\mu$-Agonisten wie Morphin, Behandlung von Entzugserscheinungen, Reduzierung des Suchtpotentials von Morphinen, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, aterieller Blutdruck, Wasserspeicher-Krankheiten, intestinale Motilität (Diarrhoe), relaxierende Effekte auf die Atemwege, Mikturations Reflex (Haminkontinenz). Weiter wird die Verwendung von Agonisten und Antagonisten als Anoretika, Analgetika (auch in Coadministration mit Opioiden) oder Nootropika diskutiert.

Entsprechend vielfältig sind die Anwendungsmöglichkeiten von Verbindungen, die an den ORL1-Rezeptor binden und diesen aktivieren oder inhibieren.

Aufgabe der vorliegenden Erfindung war es, Arzneimittel zur Verfügung zu stellen, die auf das Nociceptin/ORL1-Rezeptor-System wirken und damit für Arzneimittel insbesondere zur Behandlung der verschiedenen mit diesem System nach dem Stand der Technik in Verbindung stehenden Krankheiten bzw. zum Einsatz in den dort genannten Indikationen geeignet sind.

Ein Gegenstand der Erfindung sind daher substituierte 2-Pyridin-Cyclohexan-1,4-diaminderivate der allgemeinen Formel I,

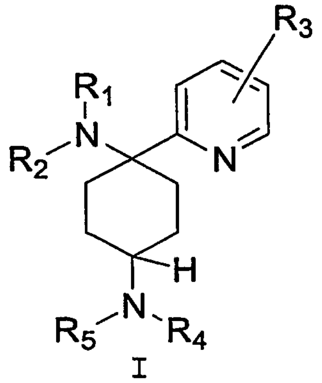

I

worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^3$ ausgewählt ist aus H; $C_{1-8}$-Alkyl, jeweils gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; SH, OH, F, Cl, 1, Br, CN, $NO_2$, $OR^{26}$, $NR^{27}R^{28}$;
mit $R^{26}$ ausgewählt aus $C_{1-6}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl oder Heteroaryl, jeweils unsubstituiert oder einfach oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH; über $C_{1-3}$-Alkyl, gesättigt oder ungesättigt, gebundenes Aryl, $C_{3-8}$-Cycloalkyl oder Heteroaryl, jeweils unsubstituiert oder ein- oder mehrfach substituiert mit F, Cl, Br, 1, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH;
mit $R^{27}$ und $R^{28}$ unabhängig voneinander ausgewählt aus H, $C_{1-6}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl oder Heteroaryl, jeweils unsubstituiert oder einfach oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4Hg$, SH und/oder OH; über $C_{1-3}$-Alkyl, gesättigt oder ungesättigt, gebundenes Aryl, $C_{3-8}$-Cycloalkyl oder Heteroaryl, jeweils unsubstituiert oder ein- oder mehrfach substituiert mit F, Cl, Br, 1, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH;
oder die Reste $R^{27}$ und $R^{28}$ bedeuten zusammen $CH_2CH_2OCH_2CH_2$, $CH_2CH_2NR^{29}CH_2CH_2$ oder $(CH_2)_{3-6}$, mit $R^{29}$ ausgewählt aus H, $C_{1-6}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl oder Heteroaryl, jeweils unsubstituiert oder einfach oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH; über $C_{1-3}$-Alkyl, gesättigt oder ungesättigt, gebundenes Aryl, $C_{3-8}$-Cycloalkyl oder Heteroaryl, jeweils

unsubstituiert oder ein- oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH;

$R^4$ ausgewählt ist aus H, $C_{1-8}$-Alkyl, gesättigt oder ungesättigt, verzweigt oder unverzweigt, einfach oder mehrfach substituiert oder unsubstituiert; oder $C(X)R^7$, $C(X)NR^7R^8$. $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}$ $CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;

$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}$- $CH_2R^{12}$, $-CHR^{11}-CH_2-CH_2R^{12}$, $-CHR^{11}-CH_2-CH_2-CH_2R^{12}$, - $C(Y)R^{12}$, -C (Y)-$CH_2R^{12}$, -C(Y)-$CH_2$-$CH_2R^{12}$ oder-C(Y)-$CH_2$-$CH_2$-$CH_2R^{12}$
mit Y = O, S oder $H_2$,

mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder C(O)O-$C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,

oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis;
in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate.

Alle diese erfindungsgemäßen Verbindungen bzw. Verbindungsgruppen zeigen hervorragende Bindung an den ORL1-Rezeptor.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder C3-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12- , C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für

C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, $CHF_2$, $CF_3$ oder $CH_2OH$ sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$, Methoxy oder Ethoxy, ersetzt sein.

Unter dem Begriff $(CH_2)_{3-6}$ ist $-CH_2-CH_2-CH_2-$, $-CH_2-CH_2-CH_2-CH_2-$, $-CH_2-CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$,$-CH_2-CH_2-$, $-CH_2-CH_2-CH_2-$ und $-CH_2-CH_2-CH_2-CH_2-$ zu verstehen, unter $(CH_2)_{4-5}$ ist$-CH_2-CH_2-CH_2-CH_2-$ und $-CH_2-CH_2-CH_2-CH_2-CH_2-$ zu verstehen.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5]thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit $R^{22}$, $OR^{22}$ einem Halogen, vorzugsweise F und/oder Cl, einem $CF_3$, einem CN, einem $NO_2$, einem $NR^{23}R^{24}$, einem $C_{1-6}$-Alkyl (gesättigt), einem $C_{1-6}$-Alkoxy, einem $C_{3-8}$-Cycloalkoxy, einem $C_{3-8}$-Cycloalkyl oder einem $C_{2-6}$-Alkylen.

Dabei steht der Rest $R^{22}$ für H, einen $C_{1-10}$-Alkyl-, vorzugsweise einen $C_{1-6}$-Alkyl-, einen Aryl- oder Heteroaryl- oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen, die Reste $R^{23}$ und $R^{24}$, gleich oder verschieden, für H, einen $C_{1-10}$-Alkyl-, vorzugsweise einen $C_{1-6}$-Alkyl-, einen Aryl-, einen Heteroaryl- oder einen über $C_{1-3}$-Alkyl, gesättigt oder ungesättigt, oder eine $C_{1-3}$-Alkylen-Gruppe gebundenen Aryl- oder Heteroaryl-Rest bedeuten, wobei diese Aryl und Heteroarylreste nicht selbst mit Aryl- oder Heteroaryl- Resten substituiert sein dürfen,

oder die Reste $R^{23}$ und $R^{24}$ bedeuten zusammen $CH_2CH_2OCH_2CH_2$, $CH_2CH_2NR^{25}CH_2CH_2$ oder $(CH_2)_{3-6}$, und der Rest $R^{25}$ für H, einen $C_{1-10}$-Alkyl-, vorzugsweise einen $C_{1-6}$-Alkyl-, einen Aryl-, oder Heteroaryl- Rest oder für einen über $C_{1-3}$-Alkyl, gesättigt oder ungesättigt, oder eine $C_{1-3}$-Alkylen-Gruppe gebundenen Aryl- oder Heteroaryl-Rest, wobei diese Aryl und Heteroarylreste nicht selbst mit Aryl- oder Heteroaryl-Resten substituiert sein dürfen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$- benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, $\alpha$-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH4^+$, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch $NH_4^+$, insbesondere aber (Mono-) oder (Di-) Natrium-, (Mono-) oder (Di-) Kalium-, Magnesium- oder Calzium-Salze.

In einer bevorzugten Ausführungsform sind die substituierten 2-Pyridin-Cyclohexan-1,4-diaminderivate so aufgebaut, daß gemäß Formel I

$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert;

oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,

mit $R^6$ ausgewählt aus H; $C_{1-8}$-Alkyl, gesättigt oder ungesättigt, verzweigt oder unverzweigt, einfach oder mehrfach substituiert oder unsubstituiert,

vorzugsweise

$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert;

oder die Reste $R^1$ und $R^2$ zusammen einen Ring bilden und $(CH_2)_{4-5}$ bedeuten,

insbesondere

$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

In einer bevorzugten Ausführungsform sind die substituierten 2-Pyridin-Cyclohexan-1,4-diaminderivate so aufgebaut, daß gemäß Formel

$R^3$ ausgewählt ist aus H; $C_{1-8}$-Alkyl, jeweils gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; SH, OH, F, Cl, 1, Br, CN, $NO_2$, $NH_2$, $OR^{26}$;

mit $R^{26}$ ausgewählt aus $C_{1-6}$-Alkyl, gesättigt oder ungesättigt, verzweigt oder unverzweigt, einfach oder mehrfach substituiert oder unsubstituiert;

vorzugsweise

$R^3$ ausgewählt ist aus H; $C_{1-6}$-Alkyl, jeweils gesättigt oder ungesättigt, verzweigt oder unverzweigt, einfach oder mehrfach substituiert oder unsubstituiert; SH, OH, F, Cl, I, Br, CN, $NO_2$, $NH_2$, $OR^{26}$;

mit $R^{26}$ ausgewählt aus $C_{1-6}$-Alkyl, gesättigt oder ungesättigt, verzweigt oder unverzweigt, einfach oder mehrfach substituiert oder unsubstituiert;

insbesondere

$R^3$ ausgewählt ist aus H.

In einer bevorzugten Ausführungsform sind die substituierten 2-Pyridin-Cyclohexan-1,4-diaminderivate so aufgebaut, daß gemäß Formel 1

$R^4$ ausgewählt ist aus H, $C(X)R^7$, $C(X)NR^7R^8$, $C(X)OR^9$, $C(X)SR^9$ oder $S(O_2)R^9$ mit X = O oder S,

vorzugsweise
$R^4$ ausgewählt ist aus H, $C(X)R^7$, $C(X)NR^7R^8$ oder $C(X)OR^9$ mit X = O,
insbesondere
$R^4$ ausgewählt ist aus H oder $C(O)R^7$; vorzugsweise mit $R^7$ ausgewählt aus
H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

In einer bevorzugten Ausführungsform sind die substituierten 2-Pyridin-Cyclohexan-1,4-diaminderivate so aufgebaut, daß gemäß Formel I
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann, vorzugsweise mit aromatischen und/oder heteroaromatischen Ringen, wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist, vorzugsweise der von $R^4$ und $R^5$ zusammen gebildete Heterocyclus so mit zwei weiteren Ringen kondensiert ist, dass $R^4$ und $R^5$ zusammen

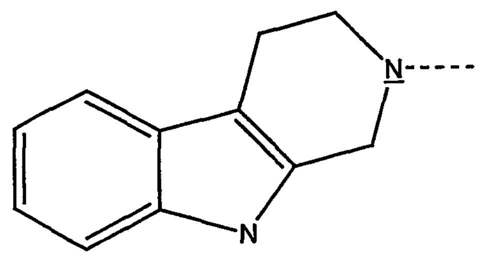

bedeuten.

In einer bevorzugten Ausführungsform sind die substituierten 2-Pyridin-Cyclohexan-1,4-diaminderivate so aufgebaut, daß gemäß Formel I
$R^4$ ausgewählt ist aus H oder $C_{1-8}$-Alkyl, gesättigt oder ungesättigt, verzweigt oder unverzweigt, einfach oder mehrfach substituiert oder unsubstituiert,
vorzugsweise
H oder $C_{1-6}$-Alkyl, gesättigt oder ungesättigt, verzweigt oder unverzweigt, einfach oder mehrfach substituiert oder unsubstituiert,
insbesondere
H oder $C_{1-3}$-Alkyl, gesättigt, unverzweigt und unsubstituiert.

In einer bevorzugten Ausführungsform sind die substituierten 2-Pyridin-Cyclohexan-1,4-diaminderivate so aufgebaut, daß gemäß Formel I
$R^5$ ausgewählt ist aus $C_{3-8}$-Cycloalkyl, Aryl oder Heteroaryl, jeweils unsubstituiert oder einfach oder mehrfach substituiert; vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Bertzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert; insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

In einer weiteren besonders bevorzugten Ausführungsform sind die substituierten 2-Pyridin-Cyclohexan-1,4-diaminderivate so aufgebaut, daß gemäß Formel I
$R^5$ ausgewählt ist aus $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
vorzugsweise
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ oder $-C(Y)-CH_2-CH_2R^{12}$
mit Y = O oder S,
insbesondere
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$ oder $-C(Y)-CH_2R^{12}$
mit Y=O.

Bezüglich dieser Ausführungsform ist es besonders bevorzugt, wenn
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert; oder $C(O)O-C_{1-2}$-Alkyl, gesättigt, unverzweigt, einfach oder mehrfach substituiert oder unsubstituiert unsubstituiert;
insbesondere
H, $CH_3$, $C_2H_5$ und $C(O)O-CH_3$.
und/oder es ist ebenso besonders bevorzugt, wenn
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyi, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

Weiter sind besonders bevorzugt die erfindungsgemäßen substituierten 2-Pyridin-Cyclohexan-1,4-diaminderivate insbesondere ausgewählt aus der folgenden Gruppe:

- ■ N-(4-Dimethylamino-4-pyridin-2-yl-cyclohexyl)-N-[2-(1 H-indol-3-yl)-ethyl]-acetamid Dihydrochlorid, unpolareres Diastereoisomer
- ■ N'-[2-(1 H-Indol-3-yl)-ethyl]-N,N-dimethyl-1 -pyridin-2-yl-cyclohexan-1,4-diamin Trihydrochlorid, unpolareres Diastereoisomer
- ■ N'-[2-(1H-Indol-3-yl)-ethyl]-N,N-dimethyl-1-pyridin-2-yl-cyclohexan-1,4-diamin Trihydrochlorid, polareres Diastereoisomer
- ■ (S)-2-(4-Dimethylamino-4-pyridin-2-yl-cyclohexylamino)-3-(1H-indol-3-yl)-propionsäuremethylester Trihydrochlorid, unpolareres Diastereoisomer
- ■ (S)-2-(4-Dimethylamino-4-pyridin-2-yl-cyclohexylamino)-3-(1 H-Indol-3-yl)-propionsäuremethylester Trihydrochlorid, polareres Diastereoisomer
- ■ (S)-2-(4-Dimethylamino-4-pyridin-2-yl-cyclohexylamino)-3-(1 H-indol-3-yl)-propionsäure Dihydrochlorid, unpolareres Diastereoisomer,

gegebenenfalls auch in Form ihrer Racemate, der genannten oder anderen reinen Stereoisomeren, insbesondere Enantiomeren oder Diastereomeren, oder in Form von Mischungen der Stereoisomeren, insbesondere der Enantiomeren

oder Diastereomeren, in einem beliebigen Mischungsverhältnis; gegebenenfalls auch in Form der Säuren oder Basen oder in Form anderer Salze, insbesondere physiologisch verträglicher Salze oder Salze physiologisch verträglicher Säuren oder Kationen; oder in Form ihrer Solvate, insbesondere der Hydrate.

Die erfindungsgemäßen Substanzen sind toxikologisch unbedenklich, so daß sie sich als pharmazeutischer Wirkstoff in Arzneimittel eignen.

Ein weiterer Gegenstand der Erfindung sind daher Arzneimittel enthaltend wenigstens ein erfindungsgemäßes substituiertes 2-Pyridin-Cyclohexan-1,4-diaminderivat, gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten 2-Pyridin-Cyclohexan-1,4-diaminderivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte 2-Pyridin-Cyclohexan-1,4-diaminderivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten 2-Pyridin-Cyclohexan-1,4-diaminderivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen substituierten 2-Pyridin-Cyclohexan-1,4-diaminderivats appliziert.

Für alle vorstehenden Formen der erfindungsgemäßen Arzneimittel ist es besonders bevorzugt, wenn das Arzneimittel neben wenigstens einem substituierten 2-Pyridin-Cyclohexan-1,4-diaminderivat noch ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes 2-Pyridin-Cyclohexan-1,4-diaminderivat als reines Diastereomer und/oder Enantiomer, als Razemat oder als nicht-äquimolare oder äquimolare Mischung der Diastereomere und/oder Enantiomere vor.

Wie in der Einleitung am Stand der Technik abzulesen, wurde der ORL1-Rezeptor insbesondere im Schmerzgeschehen identifiziert. Entsprechend können erfindungsgemäße substituierte 2-Pyridin-Cyclohexan-1,4-diaminderivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung erfindungsgemäßer substituierter 2-Pyridin-Cyclohexan-1,4-diaminderivate; gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate; zur Herstellung eines Arzneimittels zur Behandlung von Schmerz, insbesondere von akutem, neuropathischem oder chronischem Schmerz.

Wie bereits in der Einleitung ausgeführt, spielt der ORL1-Rezeptor neben der Funktion im Schmerzgeschehen noch in einer Vielzahl anderer physiologischer Prozeße insbesondere von medizinisch relevanter Bedeutung eine Rolle, so daß ein weiterer Gegenstand der Erfindung die Verwendung erfindungsgemäßer substituierter 2-Pyridin-Cyclohexan-1,4-diaminderivate, gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze oder Salze physiologisch verträglicher Säuren oder Kationen; oder in Form ihrer Solvate, insbesondere der Hydrate; zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese und/oder Anxiolyse ist.

Dabei kann es in einer der vorstehenden Verwendungen bevorzugt sein, wenn ein verwendetes substituiertes 2-Pyridin-Cyclohexan-1,4-diaminderivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Herstellung der erfindungsgemäßen substituierten 2-Pyridin-Cyclohexan-1,4-diaminderivate wie in der folgenden Beschreibung und Beispielen ausgeführt.

Besonders geeignet ist ein im folgenden Hauptverfahren A genanntes Verfahren zur Herstellung eines erfindungsgemäßen substituierten 2-Pyridin-Cyclohexan-1,4-diaminderivats gemäß Formel I mit $R^3$ = H mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

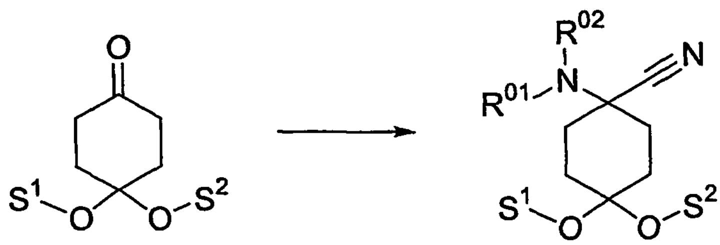

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit Cyclopentadienyl-cycloocta-1,5-diene-cobalt(I) [cpCo(cod)] zusammengebracht und unter Acetylen bestrahlt, so daß eine Verbindung gemäß Formel IVa entsteht;

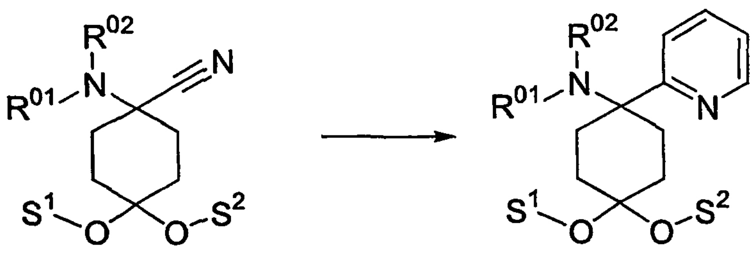

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa werden die Schutzgruppen $S^1$ und $S^2$ abgespalten, so daß ein 4-substituiertes 4-Aminocyclohexanonderivat gemäß Formel IV entsteht;

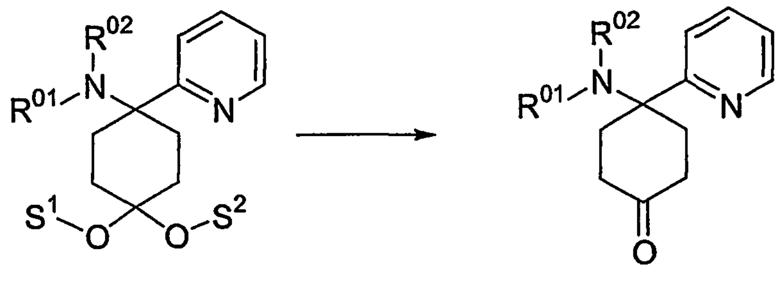

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IVa wird reduktiv mit einer Verbindung der Formel $HNR^{04}R^{05}$ aminiert, so daß ein 2-Pyridin-Cyclohexan-1,4-diaminderivat gemäß Formel V entsteht;

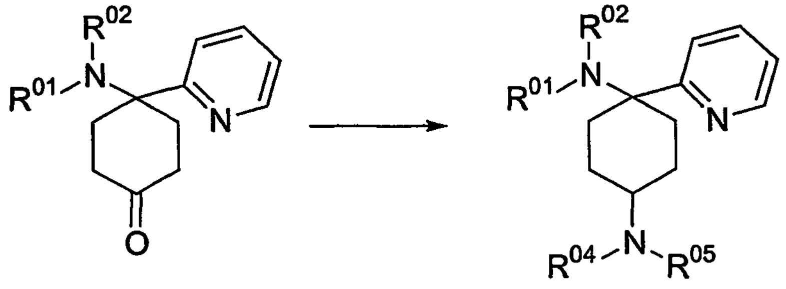

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^4$ und $R^5$ die für erfindungsgemäße Verbindungen gemäß Formel I angegebene Bedeutung haben und

$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,

mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;

$R^{04}$ ausgewählt ist aus H, mit einer Schutzgruppe versehenem H; $C_{1-8}$-Alkyl, gesättigt oder ungesättigt, verzweigt oder unverzweigt, einfach oder mehrfach substituiert oder unsubstituiert;

$R^{05}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$,$-CHR^{11}-CH_2-CH_2CH_2R^{12}$, $-C(Y)R^{12}$, $-C(Y)-CH_2R^{12}$, $-C(Y)-CH_2-CH_2R^{12}$ oder-$C(Y)-CH_2-CH_2-CH_2R^{12}$

mit $Y = H_2$,

mit $R^{11}$ ausgewählt aus

H, $C_{1-7}$-Alkyl, gesättigt oder ungesättigt, verzweigt oder unverzweigt, einfach oder mehrfach substituiert oder unsubstituiert;

und mit $R^{12}$ ausgewählt aus

H; $C_{3-8}$-Cycloalkyl, Aryl oder Heteroaryl, jeweils unsubstituiert oder einfach oder mehrfach substituiert,

oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,

und $S^1$ und $S^2$ unabhängig voneinander ausgewählt sind aus Schutzgruppen oder zusammen eine Schutzgruppe bedeuten, vorzugsweise Monoacetal.

Besonders geeignet ist ein im folgenden Alternativverfahren A genanntes Verfahren zur Herstellung eines erfindungsgemäßen substituierten 2-Pyridin-Cyclohexan-1,4-diaminderivats gemäß Formel I mit $R^3$ = H mit folgenden Schritten:

a. ein mit den Gruppen $S^1$ und $S^2$ geschütztes Cyclohexan-1,4-dion gemäß Formel 11 wird reduktiv mit einer Verbindung der Formel $HNR^{04}R^{05}$ aminiert, so daß ein 4-Aminocyclohexanonderivat gemäß Formel VI entsteht;

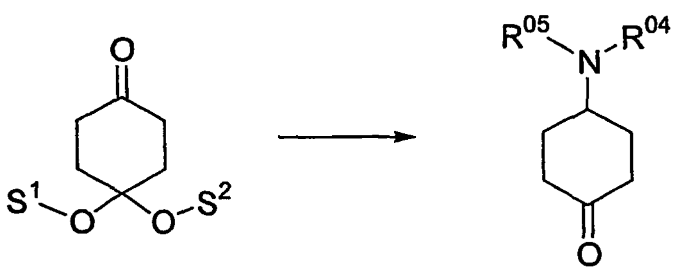

II VI

gegebenenfalls wird anschließend in beliebiger Reihenfolge und gegebenfalls wiederholt acyliert, alkyliert oder sulfoniert und/oder bei Verbindungen mit $R^{04}$ und/oder $R^{05}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{04}$ und/oder $R^{05}$ = H mindestens einmal eine Schutzgruppe eingeführt und gegebenfalls acyliert, alkyliert oder sulfoniert,

b. das 4-Aminocyclohexanonderivat gemäß Formel VI wird in Gegenwart einer Verbindung der Formel $HNR^{01}R^{02}$ mit Cyanid, vorzugsweise Kaliumcyanid, zu einem Cyclohexanonnitrilderivat der Formel VII umgesetzt,

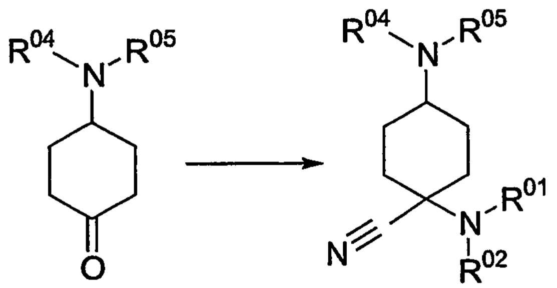

VI VII

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert,

c. das Cyclohexanonnitrilderivat der Formel VII wird mit Cyclopentadienylcycloocta-1,5-diene-cobalt(I) [cpCo(cod)] zusammengebracht und unter Acetylen bestrahlt, so daß ein 2-Pyridin-Cyclohexan-1,4-diaminderivate gemäß Formel V entsteht,

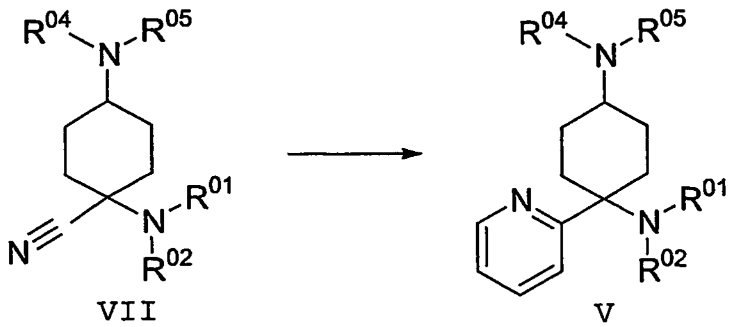

VII V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^4$ und $R^5$ die für erfindungsgemäße Verbindungen gemäß Formel I angegebene Bedeutung haben und $R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
oder die Reste $R^{01}$ und $R^{12}$ zusammen einen Ring bilden und $CH_2CH_2OCH_2CH_2$, $CH_2CH_2NR^{06}CH_2CH_2$ oder $(CH_2)_{3-6}$ bedeuten,
mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{1-8}$-Alkyl, gesättigt oder ungesättigt, verzweigt oder

unverzweigt, einfach oder mehrfach substituiert oder unsubstituiert;
$R^{05}$ ausgewählt ist aus mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit $Y = H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

Für beide Verfahren A ist es besonders günstig, wenn die Schutzgruppen am H bei $R^{01}$, $R^{02}$, $R^{04}$, $R^{05}$ und/oder $R^{06}$ ausgewählt sind aus Alkyl, Benzyl oder Carbamaten, beispielsweise FMOC, Z oder Boc.

Für das Hauptverfahren A ist es besonders günstig, wenn die reduktive Aminierung in Schritt d in Gegenwart von Amoniumformiat, Amoniumacetat oder $NaCNBH_3$ stattfindet.

Für das Hauptverfahren A ist es besonders günstig, wenn statt der reduktiven Aminierung mit $HNR^{04}R^{05}$ in Schritt d die Verbindung IV mit Hydroxylamin reagiert und nach der Oximbildung reduziert wird.

Für das Hauptverfahren A ist es besonders günstig, wenn die Bestrahlung in Schritt b zwischen 5 und 7 h dauert und/oder bei Raumtemperatur und/oder gesättigter Acetylen-Atmosphäre und/oder unter Schutzgas stattfindet.

Für das Alternativverfahren A ist es besonders günstig, wenn die Bestrahlung in Schritt c zwischen 5 und 7 h dauert und/oder bei Raumtemperatur und/oder gesättigter Acetylen-Atmosphäre und/oder unter Schutzgas stattfindet.

Geeignet ist weiter ein, im folgenden Hauptverfahren B genanntes Verfahren zur Herstellung eines erfindungsgemäßen substituierten 2-Pyridin-Cyclohexan-1,4-diaminderivats mit folgenden Schritten:

a. ein mit den Gruppen $S^1$ und $S^2$ geschütztes Cyclohexan-1,4-dion gemäß Formel 11 wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

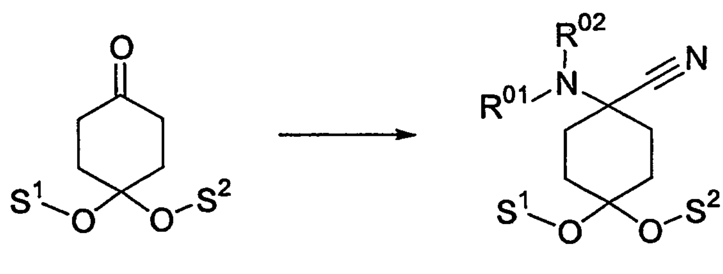

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit metallorganischen Reagenzien, bevorzugt Grignard- oder Organolithiumreagenzien, der Formel Metall-2-Pyridin-$R^3$ umgesetzt, so daß eine Verbindung gemäß Formel IVa entsteht;

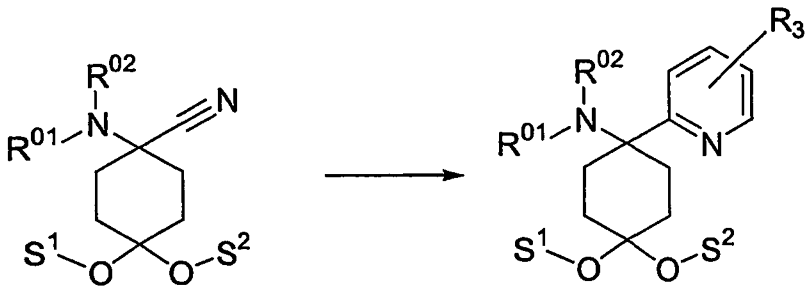

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa werden die Schutzgruppen $S^1$ und $S^2$ abgespalten, so daß ein 4-substituiertes 4-Aminocyclohexanonderivat gemäß Formel IV entsteht;

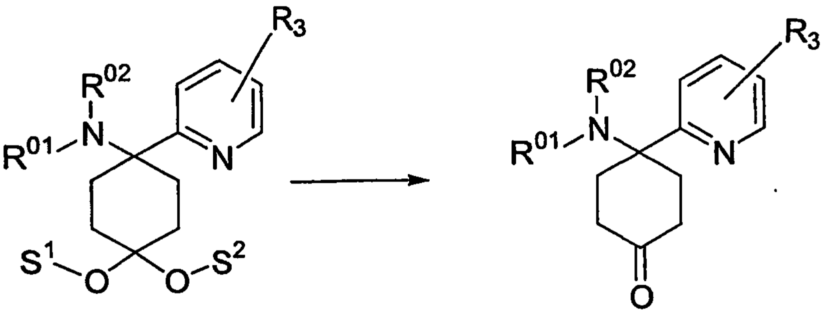

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IVa wird reduktiv mit einer Verbindung der Formel $HNR^{04}R^{05}$ aminiert, so daß ein 2-Pyridin-Cyclohexan-1,4-diaminderivat gemäß Formel V entsteht;

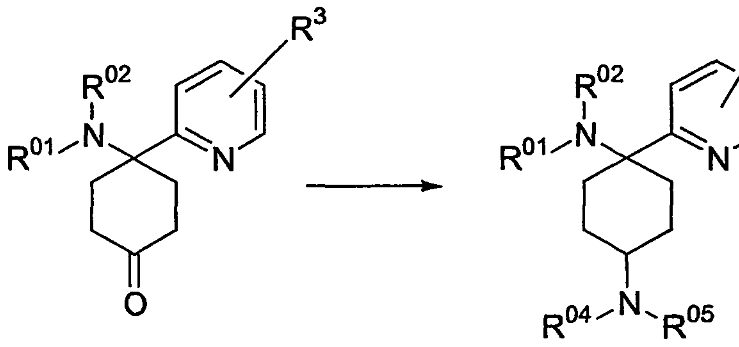

IV                                                V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel 1 entsteht,

wobei $R^1$, $R^2$, $R^3$, $R^4$ und $R^5$ die für erfindungsgemäße Verbindungen gemäß Formel 1 angegebene Bedeutung haben und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,
mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{1-8}$-Alkyl, gesättigt oder ungesättigt, verzweigt oder unverzweigt, einfach oder mehrfach substituiert oder unsubstituiert;
$R^{05}$ ausgewählt ist aus H, mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2CH_2-CH_2R^{12}$, $-C(Y)R^{12}$, $-C(Y)-CH_2R^{12}$, $-C(Y)-CH_2-CH_2R^{12}$ oder$-C(Y)-CH_2-CH_2CH_2R^{12}$
mit Y = $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert, oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

Dabei versteht man unter Alkylierung auch eine reduktive Aminierung, da sie zum gleichen Ergebnis führt.

Ein weiterer bevorzugter Gegenstand der Erfindung ist ein, im folgenden Altemativ-Verfahren B genanntes Verfahren zur Herstellung eines erfindungsgemäßen substituierten 2-Pyridin-Cyclohexan-1,4-diaminderivats mit folgenden Schritten:

a. ein mit den Gruppen $S^1$ und $S^2$ geschütztes Cyclohexan-1,4-dion gemäß Formel II wird reduktiv mit einer Verbindung der Formel $HNR^{04}R^{05}$ aminiert, so daß ein 4-Aminocyclohexanonderivat gemäß Formel VI entsteht;

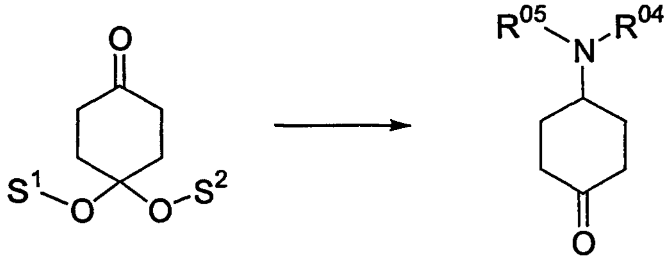

II VI

gegebenenfalls wird anschließend in beliebiger Reihenfolge und gegebenfalls wiederholt acyliert, alkyliert oder sulfoniert und/oder bei Verbindungen mit $R^{04}$ und/oder $R^{05}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{04}$ und/oder $R^{05}$ = H mindestens einmal eine Schutzgruppe eingeführt und gegebenfalls acyliert, alkyliert oder sulfoniert,

b. das 4-Aminocyclohexanonderivat gemäß Formel VI wird in Gegenwart einer Verbindung der Formel $HNR^{01}R^{02}$ mit Cyanid, vorzugsweise Kaliumcyanid, zu einem Cyclohexanonnitrilderivat der Formel VII umgesetzt,

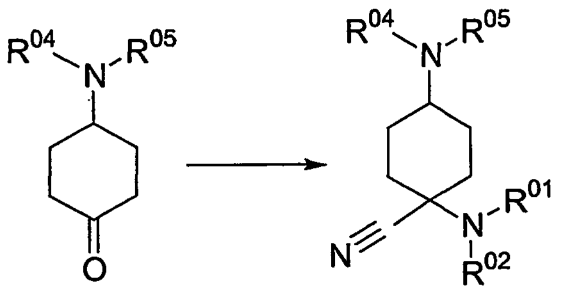

VI VII

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$= mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert,

c. das Cyclohexanonnitrilderivat der Formel VII wird mit metallorganischen Reagenzien, bevorzugt Grignard- oder Organolithiumreagenzien, der Formel Metall-2-Pyridin-$R^3$ umgesetzt und abschließend die Schutzgruppen $S^1$ und $S^2$ abgespalten, so daß ein 2-Pyridin-Cyclohexan-1,4-diaminderivate gemäß Formel V entsteht,

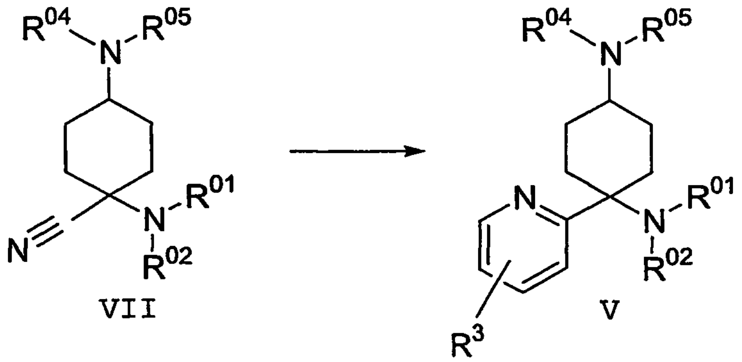

VII V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^3$, $R^4$ und $R^5$ die für die erfindungsgemäßen Verbindungen gemäß Formel I angegebene Bedeutung haben und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,
mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{1-8}$-Alkyl, gesättigt oder ungesättigt, verzweigt oder unverzweigt, einfach oder mehrfach substituiert oder unsubstituiert;
$R^{05}$ ausgewählt ist aus mit einer Schutzgruppe versehenem H; $C_{3-8}$-Cycloalkyl, Aryl oder Heteroaryl, jeweils unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$,- $C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

Für beide Verfahren B ist es bevorzugt, daß die Schutzgruppen am H bei $R^{01}$, $R^{02}$, $R^{04}$, $R^{05}$ und/oder $R^{06}$ ausgewählt sind aus Alkyl, Benzyl oder Carbamaten, beispielsweise FMOC, Z oder Boc.

Weiter ist es für das Haupt-Verfahren B bevorzugt, wenn die reduktive Aminierung in Schritt d in Gegenwart von Amoniumformiat, Amoniumacetat oder $NaCNBH_3$ stattfindet.

Für das Hauptverfahren B ist auch eine besonders günstige Ausführungsform, wenn statt der reduktiven Aminierung mit $HNR^{04}R^{05}$ in Schritt d die Verbindung IV mit Hydroxylamin reagiert und nach der Oximbildung reduziert wird.

Ebenso günstig ist es für das Anternativ-Verfahren B, wenn in Schritt b in Formel $HNR^{01}R^{02}$ der Rest $R^{01}$ = H ist, die Umsetzung mit dem Cyanid mit TMSCN geschieht und gegebenfalls anschließend eine Schutzgruppe an $R^{01}$

eingeführt wird.

Im folgenden wird die Erfindung weiter durch Beispiele erläutert.

**Beispiele**

Die folgenden Beispiele dienen zur näheren Erläuterung der Erfindung.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." bedeutet Schmelzpunkt bzw. Schmelzbereich, "RT" bedeutet Raumtemperatur, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Beispiel 1: N-(4-Dimethylamino-4-pyridin-2-yl-cyclohexyl)-N-[2-(1H-indol-3-yl)-ethyl]-acetamid Dihydrochlorid, unpolareres Diastereomer

200 g 1,4-Dioxa-spiro[4.5]decan-8-on wurden mit 200 ml Methanol, 1680 ml wäßriger Dimethylaminlösung (40 m%), 303 g Dimethylamin Hydrochlorid und 200 g Kaliumcyanid versetzt und für ca. 65 Stunden gerührt. Die erhaltene weiße Suspension wurde viermal mit je 800 ml Ether extrahiert, die vereinigten Extrakte eingeengt, der Rückstand in ca. 500 ml Dichlormethan aufgenommen und die Phasen getrennt. Die organische Phase wurde über Natriumsulfat getrocknet, filtriert und eingeengt. Es wurden 265 g 8-Dimethylamino-1,4-dioxa-spiro[4.5]decan-8-carbonitril als weißer Feststoff erhalten.

Eine Lösung aus 4,5 g 8-Dimethylamino-1,4-dioxa-spiro[4.5]decane-8-carbonitril, 50 mg Cyclopentadienyl-cycloocta-1,5-diene-cobalt(I) [cpCo(cod)] und 100 ml Toluol wurde im Schutzgas-/Acetylengegenstrom in das Reaktionsgefäß überführt. Nach Sättigung mit Acetylen wurde die Reaktionslösung unter kräftigem Rühren bei einer Temperatur von 25°C über einen Zeitraum von 6 Stunden bestrahlt. Die Reaktion wurde durch Abschalten der Lampen und Luftzufuhr unterbrochen und die Reaktionslösung eingeengt. Das erhaltene Rohprodukt (5,47 g) wurde in einem Gemisch aus Wasser (8,7 ml) und konz. Salzsäure (15 ml) aufgenommen und über Nacht bei RT gerührt. Zur Aufarbeitung wurde mit Diethylether (3 x 100 ml) gewaschen, die Phasen getrennt, die wäßrige Phase mit 32 massenprozentiger Natronlauge alkalisch gestellt, mit Dichlormethan extrahiert (3 x 100 ml), die vereinigten Extrakte getrocknet ($Na_2SO_4$), filtriert und eingeengt. Es wurden 3,72 g 4-Dimethylamino-4-pyridin-2-ylcyclohexanon erhalten.

Zu einer Lösung von 4-Dimethylamino-4-pyridin-2-yl-cyclohexanon (873 mg) und Tryptamin (640 mg) in trockenem Tetrahydrofuran (40 ml) und wasserfreiem 1,2-Dichlorethan (10 ml) wurde unter Argon Essigsäure (0,448 ml) hinzugefügt und 15 min gerührt. Nach der Zugabe von Natriumtriacetoxyborhydrid (1,2 g) wurde die Reaktionsmischung drei Tage unter Argon bei Raumtemperatur gerührt. Zur Aufarbeitung wurde das Lösungsmittels im Vakuum entfernt, der Rückstandes in 1 N Natronlauge (40 ml) und Diethylether (40 ml) aufgenommen, die Phasen getrennt, die wäßrige Phase mit Diethylether (2 x 30 ml) extrahiert, die organischen Phasen vereinigt, getrocknet und eingeengt. Das erhaltene Rohprodukt wurde durch Säulenchromatographie an Kieselgel mit Methanol und Methanol/Ammoniak (100 : 1) aufgetrennt. Das unpolarere Diastereoisomer von N'-[2-(1H-Indol-3-yl)-ethyl]-N,N-dimethyl-1-pyridin-2-yl-cyclohexan-1,4-diamin wurde als weißer Feststoff erhalten (617 mg; Smp. 150-152 °C).

N'-[2-(1H-Indol-3-yl)-ethyl]-N,N-dimethyl-1-pyridin-2-yl-cyclohexan-1,4-diamin (250 mg) wurde in trockenem Pyridin (5 ml) gelöst, mit Acetanhydrid (0,64 ml) versetzt und 22 Stunden bei Raumtemperatur gerührt. Die Reaktionsmischung wurde mit etwas Eis versetzt und dann eingeengt. Der Rückstand wurde in 1 M Natronlauge (20 ml) und Ethylacetat (20 ml) aufgenommen und gerührt. Dabei blieb ein weißer Feststoff zurück, der abgesaugt werden konnte (86 mg). Die wäßrige Phase des Filtrats wurde mit Ethylacetat (2 x 20 ml) extrahiert. Die vereinigten organischen Extrakte wurde nach dem Trocknen eingeengt. Der so erhaltene Rückstand war identisch mit dem zuvor gewonnenen Feststoff. Beide Substanzen wurden vereinigt. Es wurden 219 mg N-(4-Dimethy)amino-4-pyridin-2-yl-cyclohexyl)-N-[2-(1 H-indol-3-yl)-ethyl]-acetamid erhalten (Smp. 209-210 °C), von denen 195 mg unter leichtem Erwärmen auf 40 °C in 2-Butanon (25 ml) gelöst und mit Chlortrimethylsilan (0,303 ml) in das korrespondierende das Dihydrochlorid überführt wurden (weißer Feststoff; 219 mg; Smp. 244-247 °C).

Beispiel 2: N'-[2-(1 H-Indol-3-yl)-ethyl]-N,N-dimethyl-1-pyridin-2-yl-cyclohexan-1 ,4-diamin Trihydrochlorid, unpolareres Diastereomer

Das nach Beispiel 1 erhaltene N'-[2-(1H-Indol-3-yl)-ethyl]-N,N-dimethyl-1-pyridin-2-yl-cyclohexan-1,4-diamin (342 mg) wurde in 2-Butanon (20 ml) gelöst und mit Chlortrimethylsilan (0,59 ml) in das korrespondierende Trihydrochlorid überführt (beigefarbener Feststoff; 408 mg).

Beispiel 3: N'-[2-(1 H-Indol-3-yl)-ethyl]-N,N-dimethyl-1-pyridin-2-y1-cyclohexan-1,4-diamin Trihydrochlorid, polareres Diastereoisomer

Wie für Beispiel 1 beschrieben wurden auch 171 mg der polareren Diastereoisomers von N'-[2-(1 H-Indol-3-yl)-ethyl]-N,N-dimethyl-1-pyridin-2-yl-cyclohexan-1,4-diamin erhalten, in 2-Butanon (20 ml) gelöst und mit Chlortrimethylsilan (0,297 ml) in das korrespondierende Trihydrochlorid überführt (171 mg beiger Feststoff, Smp. 225-230 °C).

Beispiel4: N'-[2-(1H-Indol-3-yl)-ethyl]-N,N-dimethyl-1-pyridin-2-yl-cyclohexan-1,4-diamin Trihydrochlorid, unpolareres Diastereoisomer

Das Hydrochlorid von L-Tryptophanmethylesters (1,01 g) wurde mit 1,2-Dichlorethan (20 ml) und gesättigter $NaHCO_3$-Lösung (20 ml) 15 min kräftig gerührt und die wäßrige Phase mit 1,2-Dichlorethan (2 x 20 ml) extrahiert. Nach dem Trocknen mit $Na_2SO_4$ wurde die organische Phase auf 40 ml eingeengt und unter Argon mit 4-Dimethylamino-4-pyridin-2-yl-cyclohexanon (873 mg) versetzt. Zur klaren Lösung wurde Eisessig (0,448 ml) und $Na_2SO_4$ (2 g) hinzugefügt. Nach einer Reaktionszeit von 15 min wurde die Reaktionsmischung mit $NaBH(OAc)_3$ (1,2 g) versetzt und vier Tage bei Raumtemperatur gerührt. Zur Aufarbeitung wurde die Mischung mit gesättigter $NaHCO_3$-Lösung (40 ml) versetzt und 15 min gerührt. Die wäßrige Phase wurde mit Dichlormethan (2 x 30 ml) extrahiert und die vereinigten organischen Phasen nach dem Trocknen eingeengt, wobei ein hellbraunes Öl erhalten wurde. Die chromatographische Trennung des Substanzgemisches an Kieselgel erfolgte mit Ethylacetat/Methanol (4 : 1) und Methanol. Das unpolarere Produkt (820 mg leicht ölige Verbindung) wurde in 2-Butanon (50 ml) gelöst und mit Chlortrimethylsilan (1,22 ml) in das Trihydrochlorid überführt (719 mg weißer hygroskopischer Feststoff; $[\alpha]_D^{20}$ = 19,85 (MeOH, c = 1,33)).

Beispiel 5: (*S*)-2-(4-Dimethylamino-4-pyridin-2-yl-cyclohexylamino)-3-(1H-indol-3-yl)-propionsäuremethylester Trihydrochlorid , polareres Diastereomer

Wie für Beispiel 4 beschrieben wurden auch 284 mg der polareren Diastereoisomers von *S*)-2-(4-Dimethylamino-4-pyridin-2-yl-cyclohexylamino)-3-(1H-indol-3-yl)-propionsäuremethylester erhalten und in 2-Butanon (15 ml) gelöst mit Chlortrimethylsilan (0,43 ml) in das korrespondierende Trihydrochlorid überführt (171 mg weißer Feststoff;, Smp. 170-175 °C; $[\alpha]_D^{20}$ = 17,61 (MeOH, c = 1,45)).

Beispiel 6: (*S*)-2-(4-Dimethylamino-4-pyridin-2-yl-cyclohexylamino)-3-(1H-indol-3-yl)-propionsäure Dihydrochlorid, unpolareres Diastereomer

Zu einer Lösung des nach Beispiel 4 hergestellten unpolareren Diastereoisomers von N'-[2-(1H-Indol-3-yl)-ethyl]-N,N-dimethyl-1-pyridin-2-yl-cyclohexan-1,4-diamin Trihydrochlorid (378 mg) in Ethanol (20 ml) wurde 1,7N KOH (8,8 ml) hinzugefügt. Nach 70 Stunden wurde eingeengt, das zurückbleibende gelbe Öl in Wasser (10 ml) gelöst, die wäßrige Phase mit Ethylacetat (3 x 20 ml) gewaschen und mit 5,5N HCl (9,0 ml) versetzt. Die wäßrige Phase wurde eingeengt und der Rückstand mit Ethanol (2 x 20 ml) digeriert. Das zurückbleibende KCl wurde abgetrennt und das Filtrat eingeengt und mit Ether gewaschen. Dabei wurde das Dihydrochlorid von (S)-2-(4-Dimethylamino-4-pyridin-2-yl-cyclohexylamino)-3-(1 H-indol-3-yl)-propionsäure Dihydrochlorid, unpolareres erhalten (307 mg $[\alpha]_D^{20}$ = 20,69 (MeOH, c = 1,213)).

**Beispiel 8:**

**Messung der ORL1-Bindung**

Die Cyclohexan-1,4-diaminderivate der allgemeinen Formel I wurden in einem Rezeptorbindungsassay mit $^3H$-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von $^3H$-Nociceptin/Orphanin FQ betrug bei diesen Versuchen 0.5 nM. Die Bindungsassays wurden mit je 20 μg Membranprotein je 200 μl Ansatz in 50 mM Hepes, pH 7,4, 10 mM $MgCl_2$ und 1 mM EDTA durchgeführt. Die Bindung

an den ORL1-Rezeptor wurde unter Verwendung von je 1 mg WGA-SPA Beads (Amersham-Pharmacia, Freiburg), durch einstündige Inkubation des Ansatzes bei Raumtemperatur und anschließende Messung im Szintillationscounter Trilux (Wallac, Finnland), bestimmt. Die Affinität wird als $K_i$-Wert in μM angegeben.

| Beispiel | ORL1 Ki/μM |
|---|---|
| 1 | 0,18 |
| 2 | 0,013 |
| 3 | 0,34 |
| 4 | 0,093 |
| 5 | 0,47 |
| 6 | 0,28 |

**Beispiel 9:**

**Analgesieprüfung im Tail-Flick-Test an der Maus**

Die Mäuse wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Mäusen 3 bis 5 Sekunden betrug. Vor der Applikation der Lösungen enthaltend die erfindungsgemäße Verbindung bzw. der jeweiligen Vergleichslösungen wurden die Mäuse innerhalb von fünf Minuten zweimal vorgetestet und der Mittelwert dieser Messungen als Vortestmittelwert berechnet.

Die Lösungen der erfindungsgemäßen Verbindung der allgemeinen Formel I sowie die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$\%MPE = [(T_1 - T_0)/(T_2 - T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).

Die untersuchten erfindungsgemäßen Verbindungen zeigten eine analgetische Wirkung. Die Ergebnisse ausgewählter Untersuchungen sind in der nachfolgenden Tabelle zusammengefaßt.

**Tabelle:**

| **Beispiel Nr.** | **%MPE im Vergleich zur Kontrollgruppe** |
|---|---|
| 1 | 71 (10) |
| 4 | 91 (10) |

: In Klammem ist jeweils die Dosierung in mg/kg bei intravenöser Applikation angegeben

**Beispiel 10:**

**Parenterale Lösung eines erfindungsgemäßen substituierten 2-Pyridin-Cyclohexan-1,4-diaminderivats**

38 g eines der erfindungsgemäßen substituierten 2-Pyridin-Cyclohexan-1,4-diaminderivate, hier gemäß Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

**Patentansprüche**

**1.** Substituierte 2-Pyridin-Cyclohexan-1,4-diaminderivate der allgemeinen Formel I,

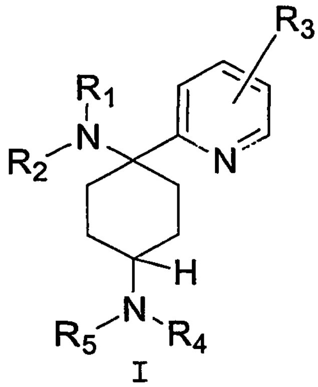

I

, worin

$R^1$ und $R^2$ unabhängig voneinander ausgewählt sind aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^3$ ausgewählt ist aus H; $C_{1-8}$-Alkyl, jeweils gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; SH, OH, F, Cl, I, Br, CN, $NO_2$, $OR^{26}$, $NR^{27}R^{28}$;
mit $R^{26}$ ausgewählt aus $C_{1-6}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl oder Heteroaryl, jeweils unsubstituiert oder einfach oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2Hs$, $OC_3H_7$, $OC_4H_9$, SH und/oder OH; über $C_{1-3}$-Alkyl, gesättigt oder ungesättigt, gebundenes Aryl, $C_{3-8}$-Cycloalkyl oder Heteroaryl, jeweils unsubstituiert oder ein- oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH;
mit $R^{27}$ und $R^{28}$ unabhängig voneinander ausgewählt aus H, $C_{1-6}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl oder Heteroaryl, jeweils unsubstituiert oder einfach oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH; über $C_{1-3}$-Alkyl, gesättigt oder ungesättigt, gebundenes Aryl, $C_{3-8}$-Cycloalkyl oder Heteroaryl, jeweils unsubstituiert oder ein- oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH; oder die Reste $R^{27}$ und $R^{28}$ bedeuten zusammen $CH_2CH_2OCH_2CH_2$, $CH_2CH_2NR^{29}CH_2CH_2$ oder $(CH_2)_{3-6}$, mit
$R^{29}$ ausgewählt aus H, $C_{1-6}$-Alkyl, gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl oder Heteroaryl, jeweils unsubstituiert oder einfach oder mehrfach substituiert mit F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH; über $C_{1-3}$-Alkyl, gesättigt oder ungesättigt, gebundenes Aryl, $C_{3-8}$-Cycloalkyl oder Heteroaryl, jeweils unsubstituiert oder ein- oder mehrfach substituiert mit F, Cl, Br, I, $NH_2$, $NO_2$, $CF_3$, $CHF_2$,

$CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH und/oder OH;
$R^4$ ausgewählt ist aus H, $C_{1-8}$-Alkyl, gesättigt oder ungesättigt, verzweigt oder unverzweigt, einfach oder mehrfach substituiert oder unsubstituiert; oder $C(X)R^7$, $C(X)NR^7R^8$, $C(X)OR^9$, $C(X)SR^9$, $S(O_2)R^9$
mit X = O oder S,
mit $R^7$ ausgewählt aus H, $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
mit $R^8$ ausgewählt aus H, $C_{1-4}$-Alkyl, gesättigt oder ungesättigt, verzweigt oder unverzweigt, einfach oder mehrfach substituiert oder unsubstituiert oder
die Reste $R^7$ und $R^8$ zusammen einen Ring bilden und $CH_2CH_2OCH_2CH_2$, $CH_2CH_2NR^{10}CH_2CH_2$ oder $(CH_2)_{3-6}$ bedeuten,
mit $R^{10}$ ausgewählt aus H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
mit $R^9$ ausgewählt aus $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl, Heteroaryl, jeweils unsubstituiert oder einfach oder mehrfach substituiert; über eine gesättigte oder ungesättigte, verzweigte oder unverzweigte, substituierte oder unsubstituierte $C_{1-4}$-Alkyl-Gruppe gebundenem Aryl, $C_{3-8}$-Cycloalkyl oder Heteroaryl, jeweils unsubstituiert oder einfach oder mehrfach substituiert;
$R^5$ ausgewählt ist aus $C_{3-8}$-Cycloalkyl, Aryl oder Heteroaryl, jeweils unsubstituiert oder einfach oder mehrfach substituiert; $-CHR^{11}R^{12}$,$-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert; oder $C(O)O-C_{1-6}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^4$ und $R^5$ zusammen einen Heterocyclus mit zwischen 3 und 8 Atomen im Ring bilden, gesättigt oder ungesättigt; einfach oder mehrfach substituiert oder unsubstituiert, der gegebenenfalls mit weiteren Ringen kondensiert sein kann,
gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer physiologisch verträglichen Salze oder Salze physiologisch verträglicher Säuren oder Kationen; oder in Form ihrer Solvate, insbesondere der Hydrate.

**2.** Substituierte 2-Pyridin-Cyclohexan-1,4-diaminderivate gemäß Anspruch 1,
**dadurch gekennzeichnet, daß**
$R^1$ und $R^2$ unabhängig voneinander ausgewählt sind aus H; $C_{1-8}$-Alkyl, gesättigt oder ungesättigt, verzweigt oder unverzweigt, einfach oder mehrfach substituiert oder unsubstituiert;
oder die Reste $R^1$ und $R^2$ zusammen einen Ring bilden und $CH_2CH_2OCH_2CH_2$, $CH_2CH_2NR^6CH_2CH_2$ oder $(CH_2)_{3-6}$ bedeuten,
mit $R^6$ ausgewählt aus H; $C_{1-8}$-Alkyl, gesättigt oder ungesättigt, verzweigt oder unverzweigt, einfach oder mehrfach substituiert oder unsubstituiert,
vorzugsweise
$R^1$ und $R^2$ unabhängig voneinander ausgewählt sind aus H; $C_{1-4}$-Alkyl, gesättigt oder ungesättigt, verzweigt oder unverzweigt, einfach oder mehrfach substituiert oder unsubstituiert;
oder die Reste $R^1$ und $R^2$ zusammen einen Ring bilden und $(CH_2)_{4-5}$ bedeuten,
insbesondere
$R^1$ und $R^2$ unabhängig voneinander ausgewählt sind aus Methyl oder Ethyl oder die Reste $R^1$ und $R^2$ zusammen einen Ring bilden und $(CH_2)_5$ bedeuten.

**3.** Substituierte 2-Pyridin-Cyclohexan-1,4-diaminderivate gemäß Anspruch 1,

**dadurch gekennzeichnet, daß**
$R^3$ ausgewählt ist aus H; $C_{1-8}$-Alkyl, jeweils gesättigt oder ungesättigt, verzweigt oder unverzweigt, einfach oder mehrfach substituiert oder unsubstituiert; $C_{3-8}$-Cycloalkyl, gesättigt oder ungesättigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert; SH, OH, F, Cl, 1, Br, CN, $NO_2$, $NH_2$, $OR^{26}$;
mit $R^{26}$ ausgewählt aus $C_{1-6}$-Alkyl, gesättigt oder ungesättigt, verzweigt oder unverzweigt, einfach oder mehrfach substituiert oder unsubstituiert;
vorzugsweise
$R^3$ ausgewählt ist aus H; $C_{1-6}$-Alkyl, jeweils gesättigt oder ungesättigt, verzweigt oder unverzweigt, einfach oder mehrfach substituiert oder unsubstituiert; SH, OH, F, Cl, I, Br, CN, $NO_2$, $NH_2$, $OR^{26}$;
mit $R^{26}$ ausgewählt aus $C_{1-6}$-Alkyl, gesättigt oder ungesättigt, verzweigt oder unverzweigt, einfach oder mehrfach substituiert oder unsubstituiert;
insbesondere
$R^3$ ausgewählt ist aus H.

4. Substituierte 2-Pyridin-Cyclohexan-1,4-diaminderivate gemäß einem der Ansprüche 1 bis 3, **dadurch gekennzeichnet, daß**
$R^4$ ausgewählt ist aus H, $C(X)R^7$, $C(X)NR^7R^8$, $C(X)OR^9$, $C(X)SR^9$ oder $S(O_2)R^9$ mit X = O oder S,
vorzugsweise
$R^4$ ausgewählt ist aus H, $C(X)R^7$, $C(X)NR^7R^8$ oder $C(X)OR^9$ mit X = O,
insbesondere
$R^4$ ausgewählt ist aus H oder $C(O)R^7$; vorzugsweise mit $R^7$ ausgewählt aus
H; oder $C_{1-8}$-Alkyl gesättigt oder ungesättigt, verzweigt oder unverzweigt, einfach oder mehrfach substituiert oder unsubstituiert;
vorzugsweise
H; oder $C_{1-3}$-Alkyl gesättigt, unsubstituiert, verzweigt oder unverzweigt;
insbesondere $CH_3$.

5. Substituierte 2-Pyridin-Cyclohexan-1,4-diaminderivate gemäß einem der Ansprüche 1 bis 3, **dadurch gekennzeichnet, daß**
$R^4$ und $R^5$ zusammen einen Heterocyclus mit zwischen 3 und 8 Atomen im Ring bilden, gesättigt oder ungesättigt; einfach oder mehrfach substituiert oder unsubstituiert, vorzugsweise mit zwischen 5 und 7 Atomen im Ring, wovon neben dem obligatorischen N 0 bis 1 weitere Heteroatome, ausgewählt aus N, S oder O, im Ring sind;
wobei der von $R^4$ und $R^5$ zusammen gebildete Heterocyclus gegebenenfalls mit weiteren Ringen kondensiert sein kann,
vorzugsweise mit aromatischen und/oder heteroaromatischen Ringen, wobei diese mit weiteren aromatischen und/oder heteroaromatischen Ringen kondensiert sein können,
insbesondere der von $R^4$ und $R^5$ zusammen gebildete Heterocyclus mit ein oder zwei weiteren Ringen kondensiert ist,
vorzugsweise der von $R^4$ und $R^5$ zusammen gebildete Heterocyclus so mit zwei weiteren Ringen kondensiert ist, dass $R^4$ und $R^5$ zusammen

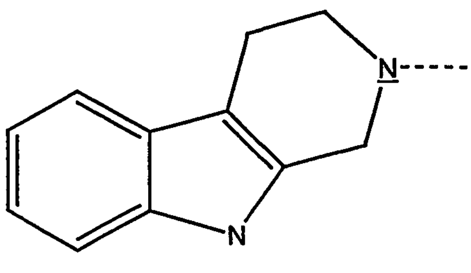

bedeuten.

6. Substituierte 2-Pyridin-Cyclohexan-1,4-diaminderivate gemäß einem der Ansprüche 1 bis 3, **dadurch gekennzeichnet, daß**
$R^4$ ausgewählt ist aus H oder $C_{1-8}$-Alkyl, gesättigt oder ungesättigt, verzweigt oder unverzweigt, einfach oder mehrfach substituiert oder unsubstituiert,
vorzugsweise
H oder $C_{1-6}$-Alkyl, gesättigt oder ungesättigt, verzweigt oder unverzweigt, einfach oder mehrfach substituiert oder unsubstituiert,
insbesondere
H oder $C_{1-3}$-Alkyl, gesättigt, unverzweigt und unsubstituiert.

**7.** Substituierte 2-Pyridin-Cyclohexan-1,4-diaminderivate gemäß einem der Ansprüche 1 bis 6, **dadurch gekennzeichnet, daß**
$R^5$ ausgewählt ist aus $C_{3-8}$-Cycloalkyl, Aryl oder Heteroaryl, jeweils unsubstituiert oder einfach oder mehrfach substituiert;
vorzugsweise
$R^5$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^5$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**8.** Substituierte 2-Pyridin-Cyclohexan-1,4-diaminderivate gemäß einem der Ansprüche 1 bis 6, **dadurch gekennzeichnet, daß**
$R^5$ ausgewählt ist aus $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = O, S oder $H_2$,
vorzugsweise
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ oder $-C(Y)-CH_2-CH_2R^{12}$
mit Y = O oder S,
insbesondere
$R^5$ ausgewählt ist aus $-CHR^{11}R^{12}$, $-CHR^{11}-CH_2R^{12}$, $-CHR^{11}-CH_2-CH_2R^{12}$, $-C(Y)R^{12}$ oder $-C(Y)-CH_2R^{12}$
mit Y = O.

**9.** Substituierte 2-Pyridin-Cyclohexan-1,4-diaminderivate gemäß Anspruch 8,
**dadurch gekennzeichnet, daß**
$R^{11}$ ausgewählt ist aus
H, $C_{1-4}$-Alkyl, gesättigt oder ungesättigt, verzweigt oder unverzweigt, einfach oder mehrfach substituiert oder unsubstituiert; oder $C(O)O-C_{1-4}$-Alkyl, gesättigt oder ungesättigt, verzweigt oder unverzweigt, einfach oder mehrfach substituiert oder unsubstituiert;
vorzugsweise
H, $C_{1-4}$-Alkyl, gesättigt oder ungesättigt, verzweigt oder unverzweigt, einfach oder mehrfach substituiert oder unsubstituiert; oder $C(O)O-C_{1-2}$-Alkyl, gesättigt, unverzweigt, einfach oder mehrfach substituiert oder unsubstituiert unsubstituiert;
insbesondere
H, $CH_3$, $C_2H_5$ und $C(O)O-CH_3$.

**10.** Substituierte2-Pyridin-Cyclohexan-1,4-diaminderivate gemäß Anspruch 8,
**dadurch gekennzeichnet, daß**
$R^{12}$ ausgewählt ist aus $C_{3-8}$-Cycloalkyl, Aryl oder Heteroaryl, jeweils unsubstituiert oder einfach oder mehrfach substituiert;
vorzugsweise
$R^{12}$ ausgewählt ist aus Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert;
insbesondere
$R^{12}$ ausgewählt ist aus Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyridyl, Pyrrolyl, Pyrazinyl oder Pyrimidyl, jeweils unsubstituiert oder einfach oder mehrfach substituiert.

**11.** Substituierte 2-Pyridin-Cyclohexan-1,4-diaminderivate gemäß einem der Ansprüche 1 bis 10, **dadurch gekennzeichnet, daß** sie ausgewählt sind aus der folgenden Gruppe:

■ N-(4-Dimethylamino-4-pyridin-2-yl-cyclohexyl)-N-[2-(1H-indol-3-yl)-ethyl]-acetamid Dihydrochlorid, unpolareres Diastereoisomer
■ N'-[2-(1H-Indol-3-yl)-ethyl]-N,N-dimethyl-1-pyridin-2-yl-cyclohexan-1,4-diamin Trihydrochlorid, unpolareres Diastereoisomer
■ N'-[2-(1H-Indol-3-yl)-ethyl]-N,N-dimethyl-1-pyridin-2-yl-cyclohexan-1,4-diamin Trihydrochlorid, polareres Diastereoisomer
■ (*S*)-2-(4-Dimethylamino-4-pyridin-2-yl-cyclohexylamino)-3-(1H-indol-3-yl)-propionsäuremethylester Trihydrochlorid, unpolareres Diastereoisomer
■ (*S*)-2-(4-Dimethylamino-4-pyridin-2-yl-cyclohexylamino)-3-(1H-indol-3-yl)-propionsäuremethylester Trihydrochlorid, polareres Diastereoisomer
■ (*S*)-2-(4-Dimethylamino-4-pyridin-2-yl-cydohexylamino)-3-(1H-indol-3-yl)-propionsäure Dihydrochlorid, unpolareres Diastereoisomer,

gegebenenfalls auch in Form ihrer Racemate, der genannten oder anderen reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; gegebenenfalls auch in Form der Säuren oder Basen oder in Form anderer Salze, insbesondere physiologisch verträglicher Salze oder Salze physiologisch verträglicher Säuren oder Kationen; oder in Form ihrer Solvate, insbesondere der Hydrate.

**12.** Arzneimittel enthaltend mindestens ein substituiertes 2-Pyridin-Cyclohexan-1,4-diaminderivat gemäß einem der Ansprüche 1 bis 11, gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weitere Wirkstoffe.

**13.** Arzneimittel gemäß Anspruch 12, **dadurch gekennzeichnet, daß** das Arzneimittel neben wenigstens einem substituierten 2-Pyridin-Cyclohexan-1,4-diaminderivat noch ein Opioid, vorzugsweise ein starkes Opioid, insbesondere Morphin, oder ein Anesthetikum, vorzugsweise Hexobarbital oder Halothan, enthält.

**14.** Verwendung eines substituierten 2-Pyridin-Cyclohexan-1,4-diaminderivats gemäß gemäß einem der Ansprüche 1 bis 11 gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem neuropathischem oder chronischem Schmerz.

**15.** Verwendung eines substituierten 2-Pyridin-Cyclohexan-1,4-diaminderivats gemäß gemäß einem der Ansprüche 1 bis 11 gegebenenfalls in Form seines Racemats, der reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form seiner Säuren oder seiner Basen oder in Form seiner physiologisch verträglichen Salze oder Salze physiologisch verträglicher Säuren oder Kationen; oder in Form seiner Solvate, insbesondere der Hydrate; zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Schwierigkeiten (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Haminkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese und/oder Anxiolyse.

**16.** Verfahren zur Herstellung eines substituierten 2-Pyridin-Cyclohexan-1,4-diaminderivats gemäß einem der Ansprüche 1 bis 11 mit $R^3$ = H mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

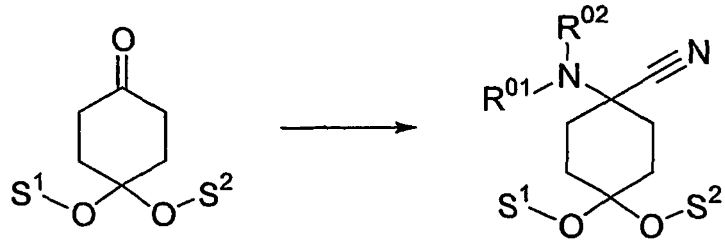

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/ oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit Cyclopentadienyl-cycloocta-1,5-diene-cobalt(1) [cpCo(cod)] zusammengebracht und unter Acetylen bestrahlt, so daß eine Verbindung gemäß Formel IVa entsteht;

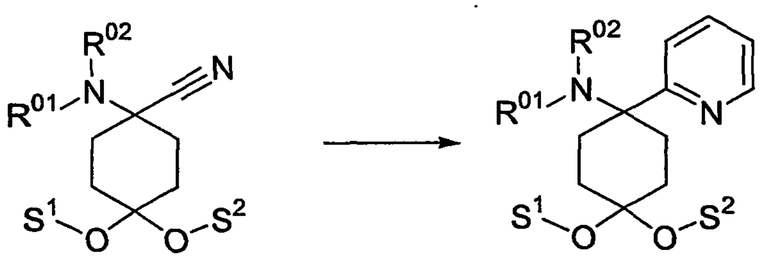

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/ oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa werden die Schutzgruppen $S^1$ und $S^2$ abgespalten, so daß ein 4-substituiertes 4-Aminocyclohexanonderivat gemäß Formel IV entsteht;

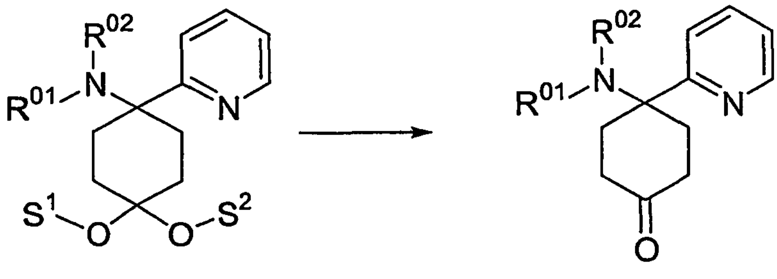

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgruppe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. das 4-substituierte 4-Aminocyclohexanonderivat gemäß Formel IVa wird reduktiv mit einer Verbindung der Formel $HNR^{04}R^{05}$ aminiert, so daß ein 2-Pyridin-Cyclohexan-1,4-diaminderivat gemäß Formel V entsteht;

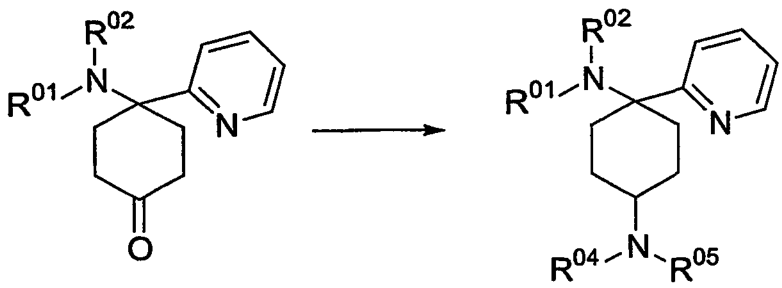

IV V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^4$ und $R^5$ die in Anspruch 1 angegebene Bedeutung haben
und
$R^{01}$ und $R^{02}$ unabhängig voneinander ausgewählt sind aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
oder die Reste $R^{01}$ und $R^{02}$ zusammen einen Ring bilden und $CH_2CH_2OCH_2CH_2$, $CH_2CH_2NR^{06}CH_2CH_2$ oder $(CH_2)_{3-6}$ bedeuten,
mit $R^{06}$ ausgewählt aus H; mit einer Schutzgruppe versehenem H; $C_{1-8}$-Alkyl oder $C_{3-8}$-Cycloalkyl, jeweils gesättigt oder ungesättigt, verzweigt oder unverzweigt, einfach oder mehrfach substituiert oder unsubstituiert; Aryl-, oder Heteroaryl, jeweils einfach oder mehrfach substituiert oder unsubstituiert; oder über $C_{1-3}$-Alkylen gebundenem Aryl, $C_{3-8}$-Cycloalkyl oder Heteroaryl, jeweils einfach oder mehrfach substituiert oder unsubstituiert;
$R^{04}$ ausgewählt ist aus H, mit einer Schutzgruppe versehenem H; $C_{1-8}$-Alkyl, gesättigt oder ungesättigt, verzweigt oder unverzweigt, einfach oder mehrfach substituiert oder unsubstituiert;
$R^{05}$ ausgewählt ist aus H, mit einer Schutzgruppe versehenem H; $C_{3-8}$-Cycloalkyl, Aryl oder Heteroaryl, jeweils

unsubstituiert oder einfach oder mehrfach substituiert; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ oder $-C(Y)-CH_2-CH_2-CH_2R^{12}$
mit Y = $H_2$,
mit $R^{11}$ ausgewählt aus
H, $C_{1-7}$-Alkyl, gesättigt oder ungesättigt, verzweigt oder unverzweigt, einfach oder mehrfach substituiert oder unsubstituiert;
und mit $R^{12}$ ausgewählt aus
H; $C_{3-8}$-Cycloalkyl, Aryl oder Heteroaryl, jeweils unsubstituiert oder einfach oder mehrfach substituiert,
oder $R^{04}$ und $R^{05}$ zusammen einen Heterocyclus mit zwischen 3 und 8 Atomen im Ring bilden, gesättigt oder ungesättigt; einfach oder mehrfach substituiert oder unsubstituiert,
und $S^1$ und $S^2$ unabhängig voneinander ausgewählt sind aus Schutzgruppen oder zusammen eine Schutzgruppe bedeuten, vorzugsweise Monoacetal.

**17.** Verfahren zur Herstellung eines substituierten 2-Pyridin-Cyclohexan-1,4-diaminderivats gemäß einem der Ansprüche 1 bis 11 mit $R^3$ = H mit folgenden Schritten:

a. ein mit den Gruppen $S^1$ und $S^2$ geschütztes Cyclohexan-1,4-dion gemäß Formel II wird reduktiv mit einer Verbindung der Formel $HNR^{04}R^{05}$ aminiert, so daß ein 4-Aminocyclohexanonderivat gemäß Formel VI entsteht;

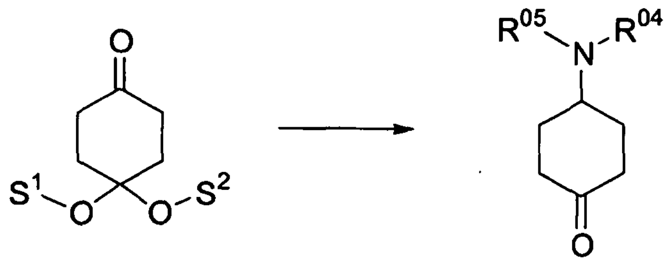

II VI

gegebenenfalls wird anschließend in beliebiger Reihenfolge und gegebenfalls wiederholt acyliert, alkyliert oder sulfoniert und/oder bei Verbindungen mit $R^{04}$ und/oder $R^{05}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{04}$ und/oder $R^{05}$ = H mindestens einmal eine Schutzgruppe eingeführt und gegebenfalls acyliert, alkyliert oder sulfoniert,

b. das 4-Aminocyclohexanonderivat gemäß Formel VI wird in Gegenwart einer Verbindung der Formel $HNR^{01}R^{02}$ mit Cyanid, vorzugsweise Kaliumcyanid, zu einem Cyclohexanonnitrilderivat der Formel VII umgesetzt,

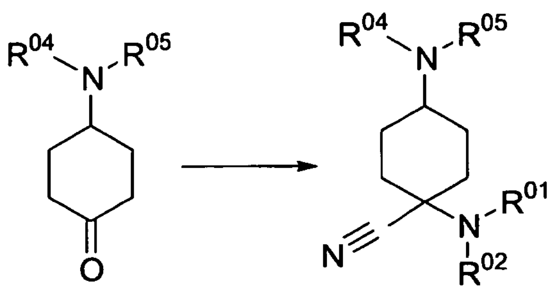

VI VII

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert,

alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert,
c. das Cyclohexanonnitrilderivat der Formel VII wird mit Cyclopentadienylcycloocta-1,5-diene-cobalt(I) [cpCo (cod)] zusammengebracht und unter Acetylen bestrahlt, so daß ein 2-Pyridin-Cyclohexan-1,4-diaminderivate gemäß Formel V entsteht,

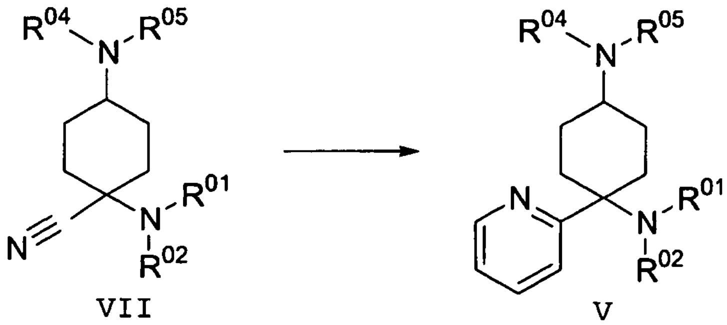

VII V

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert, bis eine Verbindung gemäß Formel I entsteht,

wobei $R^1$, $R^2$, $R^4$ und $R^5$ die in Anspruch 1 angegebene Bedeutung haben
und und die Reste $R^{01}$, $R^{02}$, $R^{06}$, $R^{04}$, $R^{05}$, $R^{11}$, $R^{12}$, $S^1$ und $S^2$ die in Anspruch 16 angegebene Bedeutung haben.

**18.** Verfahren zur Herstellung eines substituierten 2-Pyridin-Cyclohexan-1,4-diaminderivats gemäß einem der Ansprüche 16 oder 17, **dadurch gekennzeichnet, daß** die Schutzgruppen am H bei $R^{01}$, $R^{02}$, $R^{04}$, $R^{05}$ und/oder $R^{06}$ ausgewählt sind aus Alkyl, Benzyl oder Carbamaten, beispielsweise FMOC, Z oder Boc.

**19.** Verfahren zur Herstellung eines substituierten 2-Pyridin-Cyclohexan-1,4-diaminderivats gemäß Anspruch 16, **dadurch gekennzeichnet, daß** die reduktive Aminierung in Schritt d in Gegenwart von Amoniumformiat, Amoniumacetat oder $NaCNBH_3$ stattfindet.

**20.** Verfahren zur Herstellung eines substituierten 2-Pyridin-Cyclohexan-1,4-diaminderivats gemäß einem der Ansprüche 1 bis 11 mit $R^3$ = H mit folgenden Schritten:

a. ein mit den Gruppen $S^1$ und $S^2$ geschütztes Cyclohexan-1,4-dion gemäß Formel II wird in Gegenwart einer Verbindung der Formel $HNR^{01}R^{02}$ mit einem Cyanid, vorzugsweise Kaliumcyanid, zu einem geschützten N-substituierten 1-Amino-4-oxo-cyclohexancarbonitrilderivat gemäß Formel III umgesetzt;

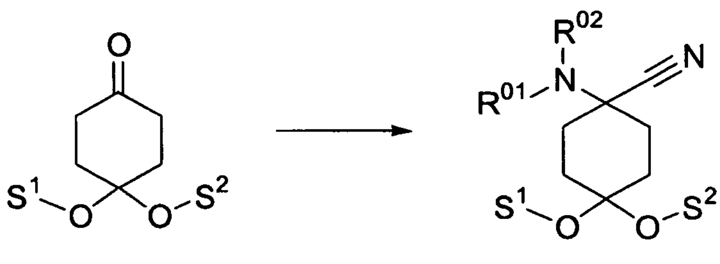

II III

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

b. das Aminonitril gemäß Formel III wird mit Cyclopentadienyl-cycloocta-1,5-diene-cobalt(1) [cpCo(cod)] zusammengebracht und unter Acetylen bestrahlt, so daß eine Verbindung gemäß Formel IVa entsteht;

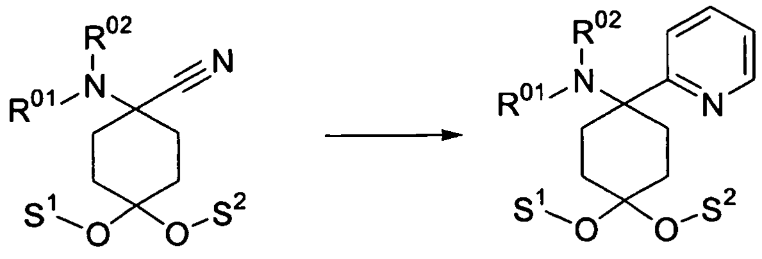

III IVa

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

c. an der Verbindung gemäß Formel IVa werden die Schutzgruppen $S^1$ und $S^2$ abgespalten, so daß ein 4-substituiertes 4-Aminocyclohexanonderivat gemäß Formel IV entsteht;

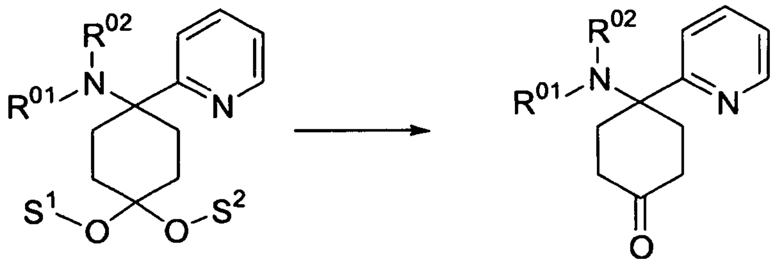

IVa IV

gegebenenfalls wird anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{06}$ = H mindestens einmal eine Schutzgruppe eingeführt und gegebenfalls acyliert, alkyliert oder sulfoniert,

d. die Verbindung IV mit Hydroxylamin reagiert und nach der Oximbildung reduziert wird,

gegebenenfalls anschließend in beliebiger Reihenfolge und gegebenfalls wiederholt acyliert, alkyliert oder sulfoniert und/oder bei Verbindungen mit $R^{01}$ und/oder $R^{02}$ und/oder $R^{04}$ und/oder $R^{05}$ und/oder $R^{06}$ = mit einer Schutzgruppe geschütztem H, mindestens einmal eine Schutzgrupe abgespalten und gegebenfalls acyliert, alkyliert oder sulfoniert und/oder bei einer Verbindungen mit $R^{01}$ und/oder $R^{02}$ und/oder $R^{04}$ und/oder $R^{05}$ und/oder $R^{06}$ = H mindestens einmal eine Schutzgruppe eingeführt und gegebenfalls acyliert, alkyliert oder sulfoniert wird, bis eine Verbindung gemäß Formel I entsteht,

wobei $R^1$, $R^2$, $R^4$ und $R^5$ die in Anspruch 1 angegebene Bedeutung haben

und die Reste $R^{01}$, $R^{02}$, $R^{06}$, $R^{04}$, $R^{05}$, $R^{11}$, $R^{12}$, $S^1$ und $S^2$ die in Anspruch 16 angegebene Bedeutung haben.

**21.** Verfahren zur Herstellung eines substituierten 2-Pyridin-Cyclohexan-1,4-diaminderivats gemäß Anspruch 16, **dadurch gekennzeichnet, daß** die Bestrahlung in Schritt b zwischen 5 und 7 h dauert und/oder bei Raumtemperatur und/oder gesättigter Acetylen-Atmosphäre und/oder unter Schutzgas stattfindet.

**22.** Verfahren zur Herstellung eines substituierten 2-Pyridin-Cyclohexan-1,4-diaminderivats gemäß Anspruch 17, **dadurch gekennzeichnet, daß** die Bestrahlung in Schritt c zwischen 5 und 7 h dauert und/oder bei Raumtemperatur und/oder gesättigter Acetylen-Atmosphäre und/oder unter Schutzgas stattfindet.

## Claims

**1.** Substituted 2-pyridine cyclohexane-1,4-diamine derivatives having the general formula I,

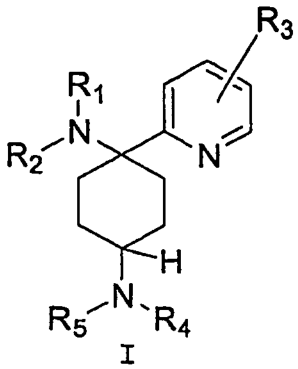

I

wherein

$R^1$ and $R^2$ are mutually independently selected from H; $C_{1-8}$ alkyl or $C_{3-8}$ cycloalkyl, each being saturated or unsaturated, branched or unbranched, mono- or polysubstituted or unsubstituted; aryl or heteroaryl, each being mono- or polysubstituted or unsubstituted; or aryl, $C_{3-8}$ cycloalkyl or heteroaryl bonded via $C_{1-3}$ alkylene, each being mono- or polysubstituted or unsubstituted;
or the radicals $R^1$ and $R^2$ together form a ring and denote $CH_2CH_2OCH_2CH_2$, $CH_2CH_2NR^6CH_2CH_2$ or $(CH_2)_{3-6}$, where $R^6$ is selected from H; $C_{1-8}$ alkyl or $C_{3-8}$ cycloalkyl, each being saturated or unsaturated, branched or unbranched, mono- or polysubstituted or unsubstituted; aryl or heteroaryl, each being mono- or polysubstituted or unsubstituted; or aryl, $C_{3-8}$ cycloalkyl or heteroaryl bonded via $C_{1-3}$ alkylene, each being mono- or polysubstituted or unsubstituted;
$R^3$ is selected from H; $C_{1-8}$ alkyl, each being saturated or unsaturated, branched or unbranched, mono- or polysubstituted or unsubstituted; $C_{3-8}$ cycloalkyl, saturated or unsaturated, mono- or polysubstituted or unsubstituted; aryl or heteroaryl, each being mono- or polysubstituted or unsubstituted; or aryl, $C_{3-8}$ cycloalkyl or heteroaryl bonded via $C_{1-3}$ alkylene, each being mono- or polysubstituted or unsubstituted; SH, OH, F, Cl, I, Br, CN, $NO_2$, $OR^{26}$, $NR^{27}R^{28}$;
where $R^{26}$ is selected from $C_{1-6}$ alkyl, saturated or unsaturated, branched or unbranched, mono- or polysubstituted or unsubstituted; $C_{3-8}$ cycloalkyl, saturated or unsaturated, mono- or polysubstituted or unsubstituted; aryl or heteroaryl, each being unsubstituted or mono- or polysubstituted with F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH and/or OH; aryl, $C_{3-8}$ cycloalkyl or heteroaryl bonded via $C_{1-3}$ alkyl, saturated or unsaturated, each being unsubstituted or mono- or polysubstituted with F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH and/or OH;
where $R^{27}$ and $R^{28}$ are mutually independently selected from H, $C_{1-6}$ alkyl, saturated or unsaturated, branched or unbranched, mono- or polysubstituted or unsubstituted; $C_{3-8}$ cycloalkyl, saturated or unsaturated, mono- or polysubstituted or unsubstituted; aryl or heteroaryl, each being unsubstituted or mono- or polysubstituted with F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH and/or OH; aryl, $C_{3-8}$ cycloalkyl or heteroaryl bonded via $C_{1-3}$ alkyl, saturated or unsaturated, each being unsubstituted or mono- or polysubstituted with F, Cl, Br, I, $NH_2$, $NO_2$, $CF_3$, $CHF_2$, $CH_2F$, $CH_3$, $C_2H_5$,

$C_3H_7$, $C_4H_9$, $OCF_3$, $OCHF_2$, $OCH_2F$, $OCH_3$, $OC_2H_5$, $OC_3H_7$, $OC_4H_9$, SH and/or OH;
or the radicals $R^{27}$ and $R^{28}$ together denote $CH_2CH_2OCH_2CH_2$, $CH_2CH_2NR^{29}CH_2CH_2$ or $(CH_2)_{3-6}$, where $R^{29}$ is selected from H, $C_{1-6}$ alkyl, saturated or unsaturated, branched or unbranched, mono- or polysubstituted or unsubstituted; $C_{3-8}$ cycloalkyl, saturated or unsaturated, mono- or polysubstituted or unsubstituted; aryl or heteroaryl, each being unsubstituted or mono- or polysubstituted with F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH and/or OH; aryl, $C_{3-8}$ cycloalkyl or heteroaryl bonded via $C_{1-3}$ alkyl, saturated or unsaturated, each being unsubstituted or mono- or polysubstituted with F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH and/or OH;
$R^4$ is selected from H, $C_{1-8}$ alkyl, saturated or unsaturated, branched or unbranched, mono- or polysubstituted or unsubstituted; or $C(X)R^7$, $C(X)NR^7R^8$, C (X) $OR^9$, C(X) $SR^9$, $S(O_2)R^9$
where X = O or S,
where $R^7$ is selected from H, $C_{1-8}$ alkyl or $C_{3-8}$ cycloalkyl, each being saturated or unsaturated, branched or unbranched, mono- or polysubstituted or unsubstituted; aryl, heteroaryl, each being unsubstituted or mono- or polysubstituted; aryl, $C_{3-8}$ cycloalkyl or heteroaryl bonded via a saturated or unsaturated, branched or unbranched, substituted or unsubstituted $C_{1-4}$ alkyl group, each being unsubstituted or mono- or polysubstituted;
where $R^8$ is selected from H, $C_{1-4}$ alkyl, saturated or unsaturated, branched or unbranched, mono- or polysubstituted or unsubstituted or
the radicals $R^7$ and $R^8$ together form a ring and denote $CH_2CH_2OCH_2CH_2$, $CH_2CH_2NR^{10}CH_2CH_2$ or $(CH_2)_{3-6}$, where $R^{10}$ is selected from H; $C_{1-8}$ alkyl or $C_{3-8}$ cycloalkyl, each being saturated or unsaturated, branched or unbranched, mono- or polysubstituted or unsubstituted; aryl or heteroaryl, each being mono- or polysubstituted or unsubstituted; or aryl, $C_{3-8}$ cycloalkyl or heteroaryl bonded via $C_{1-3}$ alkylene, each being mono- or polysubstituted or unsubstituted;
where $R^9$ is selected from $C_{1-8}$ alkyl or $C_{3-8}$ cycloalkyl, each being saturated or unsaturated, branched or unbranched, mono- or polysubstituted or unsubstituted; aryl, heteroaryl, each being unsubstituted or mono-or polysubstituted; aryl, $C_{3-8}$ cycloalkyl or heteroaryl bonded via a saturated or unsaturated, branched or unbranched, substituted or unsubstituted $C_{1-4}$ alkyl group, each being unsubstituted or mono- or polysubstituted;
$R^5$ is selected from $C_{3-8}$ cycloalkyl, aryl or heteroaryl, each being unsubstituted or mono- or polysubstituted; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, - C (Y) $-CH_2-CH_2R^{12}$ or $-C(Y)-CH_2-CH_2-CH_2R^{12}$
where Y = O, S or $H_2$,
where $R^{11}$ is selected from
H, $C_{1-7}$ alkyl, saturated or unsaturated, branched or unbranched, mono- or polysubstituted or unsubstituted; or $C(O)O-C_{1-6}$ alkyl, saturated or unsaturated, branched or unbranched, mono- or polysubstituted or unsubstituted;
and where $R^{12}$ is selected from
H; $C_{3-8}$ cycloalkyl, aryl or heteroaryl, each being unsubstituted or mono- or polysubstituted,
or $R^4$ and $R^5$ together form a heterocyclic compound with between 3 and 8 atoms in the ring, saturated or unsaturated; mono- or polysubstituted or unsubstituted, which can optionally be condensed with other rings,
optionally in the form of their racemates, their pure stereoisomers, in particular enantiomers or diastereomers, or in the form of mixtures of stereoisomers, in particular enantiomers or diastereomers, in any mixing ratio; in the form described or in the form of their acids or their bases or in the form of their physiologically compatible salts or salts of physiologically compatible acids or cations; or in the form of their solvates, in particular hydrates.

2. Substituted 2-pyridine cyclohexane-1,4-diamine derivatives according to claim 1, **characterised in that**
$R^1$ and $R^2$ are mutually independently selected from H; $C_{1-8}$ alkyl, saturated or unsaturated, branched or unbranched, mono- or polysubstituted or unsubstituted;
or the radicals $R^1$ and $R^2$ together form a ring and denote $CH_2CH_2OCH_2CH_2$, $CH_2CH_2NR^6CH_2CH_2$ or $(CH_2)_{3-6}$, where $R^6$ is selected from H; $C_{1-8}$ alkyl, saturated or unsaturated, branched or unbranched, mono- or polysubstituted or unsubstituted,
preferably
$R^1$ and $R^2$ are mutually independently selected from H; $C_{1-4}$ alkyl, saturated or unsaturated, branched or unbranched, mono- or polysubstituted or unsubstituted;
or the radicals $R^1$ and $R^2$ together form a ring and denote $(CH_2)_{4-5}$,
in particular
$R^1$ and $R^2$ are mutually independently selected from methyl or ethyl or the radicals $R^1$ and $R^2$ together form a ring and denote $(CH_2)_5$.

3. Substituted 2-pyridine cyclohexane-1,4-diamine derivatives according to claim 1, **characterised in that**

$R^3$ is selected from H; $C_{1-8}$ alkyl, each being saturated or unsaturated, branched or unbranched, mono- or polysubstituted or unsubstituted; $C_{3-8}$ cycloalkyl, saturated or unsaturated, mono-or polysubstituted or unsubstituted; aryl or heteroaryl, each being mono- or polysubstituted or unsubstituted; or aryl, $C_{3-8}$ cycloalkyl or heteroaryl bonded via $C_{1-3}$ alkylene, each being mono- or polysubstituted or unsubstituted; SH, OH, F, Cl, I, Br, CN, $NO_2$, $NH_2$, $OR^{26}$; where $R^{26}$ is selected from $C_{1-6}$ alkyl, saturated or unsaturated, branched or unbranched, mono- or polysubstituted or unsubstituted;
preferably
$R^3$ is selected from H; $C_{1-6}$ alkyl, each being saturated or unsaturated, branched or unbranched, mono- or polysubstituted or unsubstituted; SH, OH, F, Cl, I, Br, CN, $NO_2$, $NH_2$, $OR^{26}$; where $R^{26}$ is selected from $C_{1-6}$ alkyl, saturated or unsaturated, branched or unbranched, mono- or polysubstituted or unsubstituted;
in particular
$R^3$ is selected from H.

4. Substituted 2-pyridine cyclohexane-1,4-diamine derivatives according to one of claims 1 to 3, **characterised in that** $R^4$ is selected from H, C (X) $R^7$, $C(X)NR^7R^8$, $C(X)OR^9$, C(X) $SR^9$ or $S(O_2)R^9$ where X = O or S,
preferably
$R^4$ is selected from H, $C(X)R^7$, $C(X)NR^7R^8$ or $C(X)OR^9$ where X = O,
in particular
$R^4$ is selected from H or $C(O)R^7$; preferably with $R^7$ selected from
H; or $C_{1-8}$ alkyl, saturated or unsaturated, branched or unbranched, mono- or polysubstituted or unsubstituted;
preferably
H; or $C_{1-3}$ alkyl, saturated, unsubstituted, branched or unbranched;
in particular $CH_3$.

5. Substituted 2-pyridine cyclohexane-1,4-diamine derivatives according to one of claims 1 to 3, **characterised in that** $R^4$ and $R^5$ together form a heterocyclic compound with between 3 and 8 atoms in the ring, saturated or unsaturated; mono- or polysubstituted or unsubstituted, preferably with between 5 and 7 atoms in the ring, of which in addition to the obligatory N, 0 to 1 other heteroatoms, selected from N, S or O, are in the ring;
wherein the heterocyclic compound formed by $R^4$ and $R^5$ together can optionally be condensed with other rings, preferably with aromatic and/or heteroaromatic rings, wherein these can be condensed with other aromatic and/or heteroaromatic rings,
in particular the heterocyclic compound formed by $R^4$ and $R^5$ together is condensed with one or two other rings, the heterocyclic compound formed by $R^4$ and $R^5$ together is preferably condensed with two other rings in such a way that $R^4$ and $R^5$ together denote

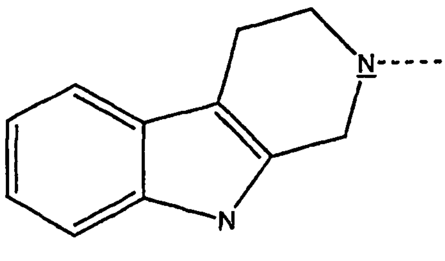

6. Substituted 2-pyridine cyclohexane-1,4-diamine derivatives according to one of claims 1 to 3, **characterised in that** $R^4$ is selected from H or $C_{1-8}$ alkyl, saturated or unsaturated, branched or unbranched, mono- or polysubstituted or unsubstituted,
preferably
H or $C_{1-6}$ alkyl, saturated or unsaturated, branched or unbranched, mono- or polysubstituted or unsubstituted,
in particular
H or $C_{1-3}$ alkyl, saturated, unbranched and unsubstituted.

7. Substituted 2-pyridine cyclohexane-1,4-diamine derivatives according to one of claims 1 to 6, **characterised in that**
$R^5$ is selected from $C_{3-8}$ cycloalkyl, aryl or heteroaryl, each being unsubstituted or mono- or polysubstituted;

preferably
$R^5$ is selected from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5] thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, each being unsubstituted or mono- or polysubstituted;
in particular
$R^5$ is selected from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, each being unsubstituted or mono- or polysubstituted.

**8.** Substituted 2-pyridine cyclohexane-1,4-diamine derivatives according to one of claims 1 to 6, **characterised in that**
$R^5$ is selected from $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ or $-C(Y)-CH_2-CH_2-CH_2R^{12}$
where Y = O, S or $H_2$,
preferably
$R^5$ is selected from $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ or $-C(Y)-CH_2-CH_2R^{12}$
where Y = O or S,
in particular
$R^5$ is selected from $-CHR^{11}R^{12}$, $-CHR^{11}-CH_2R^{12}$, $-CHR^{11}-CH_2-CH_2R^{12}$, $-C(Y)R^{12}$ or $-C(Y)-CH_2R^{12}$
where Y = O.

**9.** Substituted 2-pyridine cyclohexane-1,4-diamine derivatives according to claim 8, **characterised in that**
$R^{11}$ is selected from
H, $C_{1-4}$ alkyl, saturated or unsaturated, branched or unbranched, mono- or polysubstituted or unsubstituted; or $C(O)O-C_{1-4}$ alkyl, saturated or unsaturated, branched or unbranched, mono- or polysubstituted or unsubstituted;
preferably
H, $C_{1-4}$ alkyl, saturated or unsaturated, branched or unbranched, mono- or polysubstituted or unsubstituted; or $C(O)O-C_{1-2}$ alkyl, saturated, unbranched, mono- or polysubstituted or unsubstituted;
in particular
H, $CH_3$, $C_2H_5$ and $C(O)O-CH_3$

**10.** Substituted 2-pyridine cyclohexane-1,4-diamine derivatives according to claim 8, **characterised in that**
$R^{12}$ is selected from $C_{3-8}$ cycloalkyl, aryl or heteroaryl, each being unsubstituted or mono- or polysubstituted;
preferably
$R^{12}$ is selected from cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, fluorenyl, fluoranthenyl, benzothiazolyl, benzotriazolyl or benzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, each being unsubstituted or mono- or polysubstituted;
in particular
$R^{12}$ is selected from cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyridyl, pyrrolyl, pyrazinyl or pyrimidyl, each being unsubstituted or mono- or polysubstituted.

**11.** Substituted 2-pyridine cyclohexane-1,4-diamine derivatives according to one of claims 1 to 10, **characterised in that** they are selected from the following group:

- N-(4-dimethylamino-4-pyridin-2-yl cyclohexyl)-N-[2-(1H-indol-3-yl)ethyl] acetamide dihydrochloride, non-polar diastereoisomer
- N'-[2-(1H-indol-3-yl)ethyl]-N,N-dimethyl-1-pyridin-2-yl cyclohexane-1,4-diamine trihydrochloride, non-polar diastereoisomer
- N'-[2-(1H-indol-3-yl)ethyl]-N,N-dimethyl-1-pyridin-2-yl cyclohexane-1,4-diamine trihydrochloride, polar diastereoisomer

- (*S*)-2-(4-dimethylamino-4-pyridin-2-yl cyclohexylamino)-3-(1H-indol-3-yl) methyl propionate trihydrochloride, non-polar diastereoisomer
- (*S*)-2-(4-dimethylamino-4-pyridin-2-yl cyclohexylamino)-3-(1H-indol-3-yl) methyl propionate trihydrochloride, polar diastereoisomer
- (*S*)-2-(4-dimethylamino-4-pyridin-2-yl cyclohexylamino)-3-(1H-indol-3-yl) propionic acid dihydrochloride, non-polar diastereoisomer,

optionally also in the form of their racemates, the cited or other pure stereoisomers, in particular enantiomers or diastereomers, or in the form of mixtures of stereoisomers, in particular enantiomers or diastereomers, in any mixing ratio;
optionally also in the form of acids or bases or in the form of other salts, particularly physiologically compatible salts or salts of physiologically compatible acids or cations; or in the form of their solvates, in particular hydrates.

**12.** Medicament containing at least one substituted 2-pyridine cyclohexane-1,4-diamine derivative according to one of claims 1 to 11, optionally in the form of its racemate, pure stereoisomers, in particular enantiomers or diastereomers, or in the form of mixtures of stereoisomers, in particular enantiomers or diastereomers, in any mixing ratio; in the form described or in the form of its acids or its bases or in the form of its physiologically compatible salts or salts of physiologically compatible acids or cations; or in the form of its solvates, in particular hydrates, and optionally containing suitable additives and/or auxiliary substances and/or optionally other active ingredients.

**13.** Medicament according to claim 12, **characterised in that** in addition to at least one substituted 2-pyridine cyclohexane-1,4-diamine derivative the medicament also contains an opioid, preferably a strong opioid, in particular morphine, or an anaesthetic, preferably hexobarbital or halothane.

**14.** Use of a substituted 2-pyridine cyclohexane-1,4-diamine derivative according to one of claims 1 to 11, optionally in the form of its racemate, pure stereoisomers, in particular enantiomers or diastereomers, or in the form of mixtures of stereoisomers, in particular enantiomers or diastereomers, in any mixing ratio; in the form described or in the form of its acids or its bases or in the form of its physiologically compatible salts or salts of physiologically compatible acids or cations; or in the form of its solvates, in particular hydrates; for the production of a medicament for the treatment of pain, in particular of acute, neuropathic or chronic pain.

**15.** Use of a substituted 2-pyridine cyclohexane-1,4-diamine derivative according to one of claims 1 to 11, optionally in the form of its racemate, pure stereoisomers, in particular enantiomers or diastereomers, or in the form of mixtures of stereoisomers, in particular enantiomers or diastereomers, in any mixing ratio; in the form described or in the form of its acids or its bases or in the form of its physiologically compatible salts or salts of physiologically compatible acids or cations; or in the form of its solvates, in particular hydrates; for the production of a medicament for the treatment of anxiety conditions, stress and stress-related syndromes, depression, epilepsy, Alzheimer's disease, senile dementia, general cognitive disfunctions, learning and memory difficulties (as a nootropic), withdrawal symptoms, alcohol and/or drug and/or medication abuse and/or dependency, sexual dysfunctions, cardiovascular diseases, hypotension, hypertension, tinnitus, pruritus, migraines, hearing difficulties, deficient intestinal motility, eating disorders, anorexia, obesity, locomotive disorders, diarrhoea, cachexia, urinary incontinence or as a muscle relaxant, anticonvulsive agent or anaesthetic or for coadministration in treatment with an opioid analgesic or with an anaesthetic, for diuresis or antinatriuresis and/or anxiolysis.

**16.** Process for the production of a substituted 2-pyridine cyclohexane-1,4-diamine derivative according to one of claims 1 to 11 where $R^3$ = H, comprising the following steps:

a. a cyclohexane-1,4-dione protected with groups $S^1$ and $S^2$ according to formula II is reacted in the presence of a compound having the formula $HNR^{01}R^{02}$ with a cyanide, preferably potassium cyanide, to give a protected N-substituted 1-amino-4-oxocyclohexane carbonitrile derivative according to formula III;

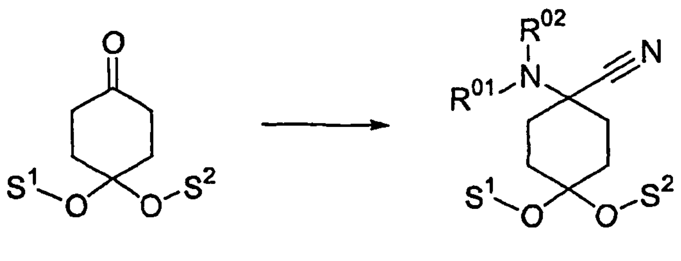

II III

acylation, alkylation or sulfonation is then optionally performed in any sequence and optionally more than once and/or in the case of compounds where $R^{01}$ and/or $R^{02}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{06}$ = H, a protective group is introduced at least once and acylation, alkylation or sulfonation optionally performed,

b. the aminonitrile according to formula III is brought into contact with cyclopentadienyl cycloocta-1,5-diene cobalt(I) [cpCo(cod)] and irradiated under acetylene, such that a compound according to formula IVa is produced;

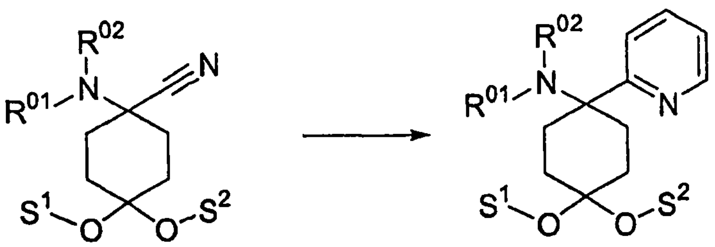

III IVa

acylation, alkylation or sulfonation is then optionally performed in any sequence and optionally more than once and/or in the case of compounds where $R^{01}$ and/or $R^{02}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{06}$ = H, a protective group is introduced at least once and acylation, alkylation or sulfonation optionally performed,

c. the protective groups $S^1$ and $S^2$ are eliminated at the compound according to formula IVa such that a tetrasubstituted 4-aminocyclohexanone derivative according to formula IV is produced;

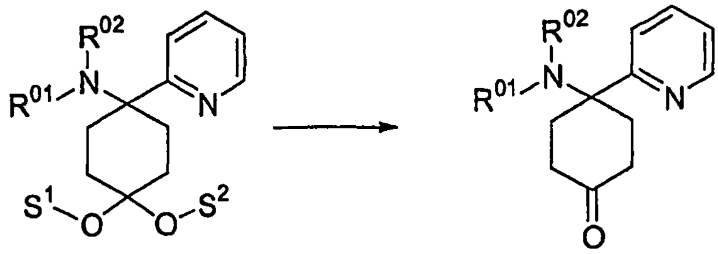

IVa IV

acylation, alkylation or sulfonation is then optionally performed in any sequence and optionally more than once and/or in the case of compounds where $R^{01}$ and/or $R^{02}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{16}$ = H, a protective group is introduced at least once and acylation, alkylation or sulfonation optionally performed,

d. the tetrasubstituted 4-aminocyclohexanone derivative according to formula IVa is reductively aminated with a compound having the formula $HNR^{04}R^{05}$ such that a 2-pyridine cyclohexane-1,4-diamine derivative according to formula V is produced;

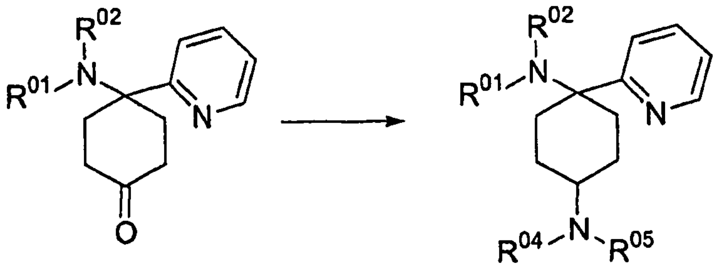

IV V

acylation, alkylation or sulfonation is then optionally performed in any sequence and optionally more than once and/or in the case of compounds

where $R^{01}$ and/or $R^{02}$ and/or $R^{04}$ and/or $R^{05}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{04}$ and/or $R^{05}$ and/or $R^{06}$ =H, a protective group is introduced at least once and acylation, alkylation or sulfonation optionally performed, until a compound according to formula I is produced, wherein $R^1$, $R^2$, $R^4$ and $R^5$ have the meaning given in claim 1
and
$R^{01}$ and $R^{02}$ are mutually independently selected from H; H provided with a protective group; $C_{1-8}$ alkyl or $C_{3-8}$ cycloalkyl, each being saturated or unsaturated, branched or unbranched, mono- or polysubstituted or unsubstituted; aryl or heteroaryl, each being mono- or polysubstituted or unsubstituted; or aryl, $C_{3-8}$ cycloalkyl or heteroaryl bonded via $C_{1-3}$ alkylene, each being mono- or polysubstituted or unsubstituted;
or the radicals $R^{01}$ and $R^{02}$ together form a ring and denote $CH_2CH_2OCH_2CH_2$, $CH_2CH_2NR^{06}CH_2CH_2$ or $(CH_2)_{3-6}$, where $R^{06}$ is selected from H; H provided with a protective group; $C_{1-8}$ alkyl or $C_{3-8}$ cycloalkyl, each being saturated or unsaturated, branched or unbranched, mono- or polysubstituted or unsubstituted; aryl or heteroaryl, each being

mono- or polysubstituted or unsubstituted; or aryl, $C_{3-8}$ cycloalkyl or heteroaryl bonded via $C_{1-3}$ alkylene, each being mono- or polysubstituted or unsubstituted;
$R^{04}$ is selected from H, H provided with a protective group; $C_{1-8}$ alkyl, saturated or unsaturated, branched or unbranched, mono- or polysubstituted or unsubstituted;
$R^{05}$ is selected from H, H provided with a protective group; $C_{3-8}$ cycloalkyl, aryl or heteroaryl, each being unsubstituted or mono- or polysubstituted; - $CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, -C (Y) $R^{12}$, -C (Y) $-CH_2R^{12}$ , C (Y) - $CH_2-CH_2R^{12}$ or -C (Y) $CH_2$- $CH_2-CH_2R^{12}$
where Y = $H_2$,
where $R^{11}$ is selected from
H, $C_{1-7}$ alkyl, saturated or unsaturated, branched or unbranched, mono- or polysubstituted or unsubstituted;
and where $R^{12}$ is selected from
H; $C_{3-8}$ cycloalkyl, aryl or heteroaryl, each being unsubstituted or mono- or polysubstituted,
or $R^{04}$ and $R^{05}$ together form a heterocyclic compound with between 3 and 8 atoms in the ring, saturated or unsaturated; mono- or polysubstituted or unsubstituted,
and $S^1$ and $S^2$ are mutually independently selected from protective groups or together denote a protective group, preferably monoacetal.

**17.** Process for the production of a substituted 2-pyridine cyclohexane-1,4-diamine derivative according to one of claims 1 to 11 where $R^3$ = H, comprising the following steps:

a. a cyclohexane-1,4-dione protected with groups $S^1$ and $S^2$ according to formula II is reductively aminated with a compound having the formula $HNR^{04}R^{05}$ such that a 4-aminocyclohexanone derivative according to formula VI is produced;

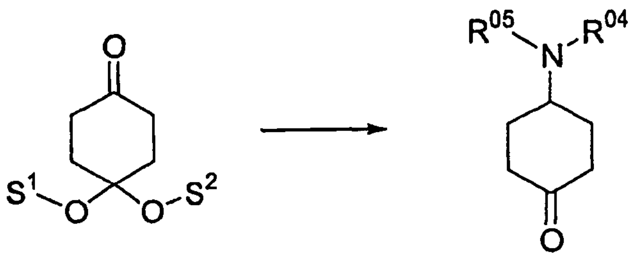

II VI

acylation, alkylation or sulfonation is then optionally performed in any sequence and optionally more than once and/or in the case of compounds where $R^{04}$ and/or $R^{05}$ = H protected by a protective group, a protective group is eliminated at least once and acylation, alkylation or sulfonation optionally performed and/or in the case of compounds where $R^{04}$ and/or $R^{05}$ = H, a protective group is introduced at least once and acylation, alkylation or sulfonation optionally performed,
b. the 4-aminocyclohexanone derivative according to formula VI is reacted in the presence of a compound having the formula $HNR^{01}R^{02}$ with cyanide, preferably potassium cyanide, to give a cyclohexanone nitrile derivative having formula VII,

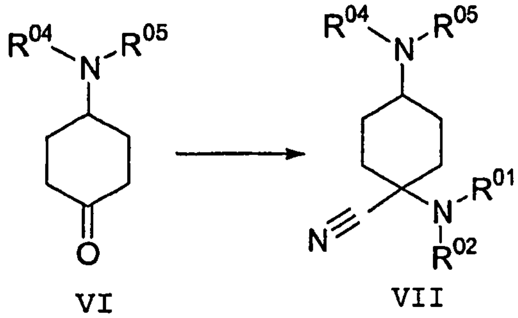

acylation, alkylation or sulfonation is then optionally performed in any sequence and optionally more than once and/or in the case of compounds where $R^{01}$ and/or $R^{02}$ and/or $R^{04}$ and/or $R^{05}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{04}$ and/or $R^{05}$ and/or $R^{06}$ = H, a protective group is introduced at least once and acylation, alkylation or sulfonation optionally performed,

c. the cyclohexanone nitrile derivative having formula VII is brought into contact with cyclopentadienyl cycloocta-1,5-diene cobalt(I) [cpCo(cod)] and irradiated under acetylene, such that a 2-pyridine cyclohexane-1,4-diamine derivative according to formula V is produced,

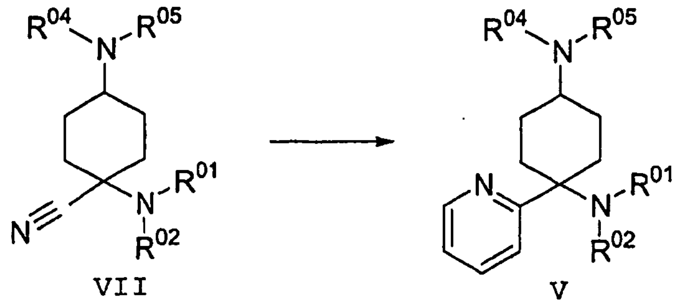

acylation, alkylation or sulfonation is then optionally performed in any sequence and optionally more than once and/or in the case of compounds where $R^{01}$ and/or $R^{02}$ and/or $R^{04}$ and/or $R^{05}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{04}$ and/or $R^{05}$ and/or $R^{06}$ = H, a protective group is introduced at least once and acylation, alkylation or sulfonation optionally performed, until a compound according to formula I is produced,

wherein $R^1$, $R^2$, $R^4$ and $R^5$ have the meaning given in claim 1

and the radicals $R^{01}$, $R^{02}$, $R^{06}$, $R^{04}$, $R^{05}$, $R^{11}$, $R^{12}$, $S^1$ and $S^2$ have the meaning given in claim 16.

**18.** Process for the production of a substituted 2-pyridine cyclohexane-1,4-diamine derivative according to one of claims 16 or 17, **characterised in that** the protective groups at H in $R^{01}$, $R^{02}$, $R^{04}$ , $R^{05}$ and/or $R^{06}$ are selected from alkyl, benzyl or carbamates, for example FMOC, Z or Boc.

**19.** Process for the production of a substituted 2-pyridine cyclohexane-1,4-diamine derivative according to claim 16, **characterised in that** the reductive amination in step d takes place in the presence of ammonium formate, ammonium acetate or $NaCNBH_3$.

**20.** Process for the production of a substituted 2-pyridine cyclohexane-1,4-diamine derivative according to one of claims 1 to 11 where $R^3$ = H, comprising the following steps:

a. a cyclohexane-1,4-dione protected with groups $S^1$ and $S^2$ according to formula II is reacted in the presence of a compound having the formula $HNR^{01}R^{02}$ with a cyanide, preferably potassium cyanide, to give a protected

N-substituted 1-amino-4-oxocyclohexane carbonitrile derivative according to formula III;

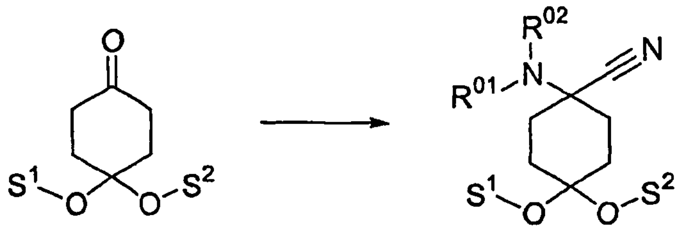

II III

acylation, alkylation or sulfonation is then optionally performed in any sequence and optionally more than once and/or in the case of compounds where $R^{01}$ and/or $R^{02}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{06}$ = H, a protective group is introduced at least once and acylation, alkylation or sulfonation optionally performed,

b. the aminonitrile according to formula III is brought into contact with cyclopentadienyl cycloocta-1,5-diene cobalt(I) [cpCo(cod)] and irradiated under acetylene, such that a compound according to formula IVa is produced;

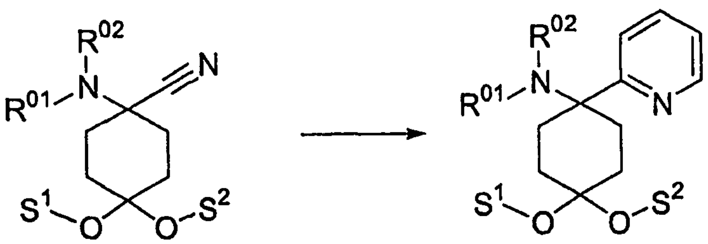

III IVa

acylation, alkylation or sulfonation is then optionally performed in any sequence and optionally more than once and/or in the case of compounds where $R^{01}$ and/or $R^{02}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{06}$ = H, a protective group is introduced at least once and acylation, alkylation or sulfonation optionally performed,

c. the protective groups $S^1$ and $S^2$ are eliminated at the compound according to formula IVa such that a tetrasubstituted 4-aminocyclohexanone derivative according to formula IV is produced;

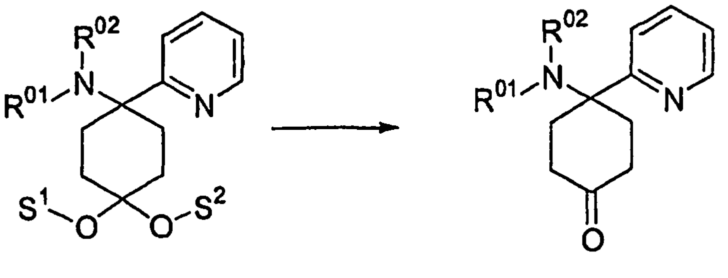

IVa IV

acylation, alkylation or sulfonation is then optionally performed in any sequence and optionally more than once and/or in the case of compounds where $R^{01}$ and/or $R^{02}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{06}$ = H, a protective group is introduced at least once and acylation, alkylation or sulfonation optionally performed,

d. compound IV is reacted with hydroxylamine and reduced after oxime formation

acylation, alkylation or sulfonation is then optionally performed in any sequence and optionally more than once and/or in the case of compounds where $R^{01}$ and/or $R^{02}$ and/or $R^{04}$ and/or $R^{05}$ and/or $R^{06}$ = H protected by a protective group, a protective group is eliminated at least once and acylation, alkylation or sulfonation optionally performed and/or in the case of compounds where $R^{01}$ and/or $R^{02}$ and/or $R^{04}$ and/or $R^{05}$ and/or $R^{06}$ = H, a protective group is introduced at least once and acylation, alkylation or sulfonation optionally performed, until a compound according to formula I is produced,

wherein $R^1$, $R^2$, $R^4$ and $R^5$ have the meaning given in claim 1

and the radicals $R^{01}$, $R^{02}$, $R^{06}$, $R^{04}$, $R^{05}$, $R^{11}$, $R^{12}$, $S^1$ and $S^2$ have the meaning given in claim 16.

**21.** Process for the production of a substituted 2-pyridine cyclohexane-1,4-diamine derivative according to claim 16, **characterised in that** the irradiation in step b lasts between 5 and 7 h and/or takes place at room temperature and/or in a saturated acetylene atmosphere and/or under protective gas.

**22.** Process for the production of a substituted 2-pyridine cyclohexane-1,4-diamine derivative according to claim 17, **characterised in that** the irradiation in step c lasts between 5 and 7 h and/or takes place at room temperature and/or in a saturated acetylene atmosphere and/or under protective gas.

## Revendications

**1.** Dérivés substitués de 2-pyridine-cyclohexane-1,4-diamine de formule générale I,

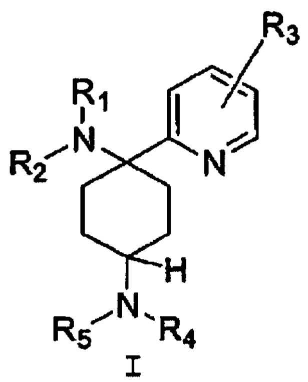

I

dans laquelle

$R^1$ et $R^2$ sont choisis, indépendamment l'un de l'autre, entre H ; un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste, lié par l'intermédiaire d'un radical alkylène en $C_1$ à $C_3$, aryle, cycloalkyle en $C_3$ à $C_8$ ou hétéroaryle, chacun substitué une ou plusieurs fois ou non substitué ;

ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,

$R^6$ étant choisi entre H ; un reste alkyle en $C_1$ à Ce ou un reste cycloalkyle en $C_3$ à $C_8$, chacun saturé ou non saturé, ramifié ou non ramifié, substitué une ou plusieurs fois ou non substitué ; un reste aryle ou un reste hétéroaryle, chacun substitué une ou plusieurs fois ou non substitué ; ou un reste, lié par l'intermédiaire d'un radical alkylène en $C_1$ à $C_3$, aryle, cycloalkyle en $C_3$ à $C_8$ ou hétéroaryle, chacun substitué une ou plusieurs fois ou non substitué ;

$R^3$ est choisi entre H ; un reste alkyle en $C_1$ à $C_8$, dans chaque cas saturé ou non saturé, ramifié ou non ramifié,

substitué une ou plusieurs fois ou non substitué ; un reste cycloalkyle en $C_3$ à $C_8$, saturé ou non saturé, substitué une ou plusieurs fois ou non substitué ; un reste aryle ou hétéroaryle, chacun substitué une ou plusieurs fois ou non substitué ; ou un reste, lié par l'intermédiaire d'un radical alkylène en $C_1$ à $C_3$, aryle, cycloalkyle en $C_3$ à Ce ou hétéroaryle, chacun substitué une ou plusieurs fois ou non substitué ; SH ; OH, F, Cl, I, Br, CN, $NO_2$, $OR^{26}$, $NR^{27}R^{28}$ ;

$R^{26}$ étant choisi entre un reste alkyle en $C_1$ à $C_6$, saturé ou non saturé, ramifié ou non ramifié, substitué une ou plusieurs fois ou non substitué ; un reste cycloalkyle en $C_3$ à $C_8$, saturé ou non saturé, substitué une ou plusieurs fois ou non substitué ; un reste aryle ou hétéroaryle, chacun non substitué ou substitué une ou plusieurs fois avec F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH et/ou OH ; ou un reste, lié par l'intermédiaire d'un radical alkyle en $C_1$ à $C_3$ saturé ou non saturé, aryle, cycloalkyle en $C_3$ à $C_8$ ou hétéroaryle, chacun non substitué ou substitué une ou plusieurs fois avec F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH et/ou OH ;

$R^{27}$ et $R^{28}$ étant choisis indépendamment l'un de l'autre entre H, un reste alkyle en $C_1$ à $C_6$, saturé ou non saturé, ramifié ou non ramifié, substitué une ou plusieurs fois ou non substitué ; un reste cycloalkyle en $C_3$ à $C_8$, saturé ou non saturé, substitué une ou plusieurs fois ou non substitué ; un reste aryle ou hétéroaryle, chacun non substitué ou substitué une ou plusieurs fois avec F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH et/ou OH ; un reste aryle, cycloalkyle en $C_3$ à $C_8$ ou hétéroaryle lié par l'intermédiaire d'un radical alkyle en $C_1$ à $C_3$ saturé ou non saturé, chacun non substitué ou substitué une ou plusieurs fois avec F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH et/ou OH ;

ou bien les restes $R^{27}$ et $R^{28}$ forment ensemble un groupe $CH_2CH_2OCH_2CH_2$, $CH_2CH_2NR^{29}CH_2CH_2$ ou $(CH_2)_{3-6}$,

$R^{29}$ étant choisi entre H, un reste alkyle en $C_1$ à $C_6$, saturé ou non saturé, ramifié ou non ramifié, substitué une ou plusieurs fois ou non substitué; un reste cycloalkyle en $C_3$ à $C_8$, saturé ou non saturé, substitué une ou plusieurs fois ou non substitué ; un reste aryle ou un reste hétéroaryle, chacun non substitué ou substitué une ou plusieurs fois avec F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH et/ou OH ; un reste aryle, cycloalkyle en $C_3$ à $C_8$ ou hétéroaryle lié par l'intermédiaire d'un radical alkyle en $C_1$ à $C_3$ saturé ou non saturé, chacun non substitué ou substitué une ou plusieurs fois avec F, Cl, Br, I, $NH_2$, $NO_2$, $CF_3$, $CHF_2$, $CH_2F$, $CH_3$, $C_2H_5$, $C_3H_7$, $C_4H_9$, $OCF_3$, $OCHF_2$, $OCH_2F$, $OCH_3$, $OC_2H_5$, $OC_3H_7$, $OC_4H_9$, SH et/ou OH ;

$R^4$ est choisi entre H, un reste alkyle en $C_1$ à $C_8$, saturé ou non saturé, ramifié ou non ramifié, substitué une ou plusieurs fois ou non substitué ; ou bien un groupe $C(X)R^7$, $C(X)NR^7R^8$, $C(X)OR^9$, $C(X)SR^9$, $S(O_2)R^9$, avec X = 0 ou S,

$R^7$ étant choisi entre H, un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste aryle, cycloalkyle en $C_3$ à $C_8$ ou hétéroaryle lié par l'intermédiaire d'un radical alkyle en $C_1$ à $C_4$ saturé ou non saturé, ramifié ou non ramifié, substitué ou non substitué, chacun non substitué ou substitué une ou plusieurs fois ;

$R^8$ étant choisi entre H ; un reste alkyle en $C_1$ à $C_4$, saturé ou non saturé, ramifié ou non ramifié, substitué une ou plusieurs fois ou non substitué, ou bien

les restes $R^7$ et $R^8$ formant ensemble un noyau et représentant un groupe $CH_2CH_2OCH_2CH_2$, $CH_2CH_2NR^{10}CH_2CH_2$ ou $(CH_2)_{3-6}$,

$R^{10}$ étant choisi entre H ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un radical alkylène en $C_1$ à $C_3$, chacun substitué une ou plusieurs ou non substitué ;

$R^9$ étant choisi entre un reste alkyle en $C_1$ à $C_8$ ou un reste cycloalkyle en $C_3$ à $C_8$, chacun saturé ou non saturé, ramifié ou non ramifié, substitué une ou plusieurs fois ou non substitué ; un reste aryle, hétéroaryle, chacun non substitué ou substitué une ou plusieurs fois ; un reste aryle, cycloalkyle en $C_3$ à $C_8$ ou hétéroaryle lié par l'intermédiaire d'un radical alkyle en $C_1$ à $C_4$ saturé ou non saturé, ramifié ou non ramifié, substitué ou non substitué, chacun non substitué ou substitué une ou plusieurs fois ;

$R^5$ est choisi entre un reste cycloalkyle en $C_3$ à $C_8$, aryle ou hétéroaryle, chacun non substitué ou substitué une ou plusieurs fois ; un groupe $-CHR^{11}R^{12}$, $-CHR^{11}-CH_2R^{12}$, $-CHR^{11}-CH_2-CH_2R^{12}$, $-CHR^{11}CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$, avec Y = O, S ou $H_2$,

$R^{11}$ étant choisi entre

H, un reste alkyle en $C_1$ à $C_7$, saturé ou non saturé, ramifié ou non ramifié, substitué une ou plusieurs fois ou

non substitué ; ou un groupe C(O)O-(alkyle en $C_1$ à $C_6$), saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois, ou bien $R^4$ et $R^5$ forment ensemble un hétérocycle ayant entre 3 et 8 atomes dans le noyau, saturé ou non saturé ; substitué une ou plusieurs fois ou non substitué, qui peut être éventuellement condensé avec d'autres noyaux,
le cas échéant sous forme de leurs racémates, de leurs stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ;

sous la forme représentée ou sous forme de leurs acides ou de leurs bases ou sous forme de leurs sels physiologiquement acceptables ou de sels d'acides ou de cations physiologiquement acceptables ; ou sous forme de leurs produits de solvatation, en particulier des hydrates.

2. Dérivés substitués de 2-pyridine-cyclohexane-1,4-diamine suivant la revendication 1, **caractérisés en ce que**
$R^1$ et $R^2$ sont choisis indépendamment l'un de l'autre entre H ; un reste alkyle en $C_1$ à $C_8$, saturé ou non saturé, ramifié ou non ramifié, substitué une ou plusieurs fois ou non substitué ;
ou bien les restes $R^1$ et $R^2$ forment ensemble un noyau et représentent $CH_2CH_2OCH_2CH_2$, $CH_2CH_2NR^6CH_2CH_2$ ou $(CH_2)_{3-6}$,
$R^6$ étant choisi entre H ; un reste alkyle en $C_1$ à $C_8$, saturé ou non saturé, ramifié ou non ramifié, substitué une ou plusieurs fois ou non substitué,
avantageusement
$R^1$ et $R^2$ sont choisis indépendamment l'un de l'autre entre H ; un reste alkyle en $C_1$ à $C_4$, saturé ou non saturé, ramifié ou non ramifié, substitué une ou plusieurs fois ou non substitué ;
ou bien les restes $R^1$ et $R^2$ forment ensemble un noyau et représentent $(CH_2)_{4-5}$,
en particulier
$R^1$ et $R^2$ sont choisis indépendamment l' un de l'autre entre un reste méthyle ou éthyle ou bien les restes $R^1$ et $R^2$ forment ensemble un noyau et représentent $(CH_2)_5$.

3. Dérivés substitués de 2-pyridine-cyclohexane-1,4-diamine suivant la revendication 1, **caractérisés en ce que**
$R^3$ est choisi entre H ; un reste alkyle en $C_1$ à Ce, à chaque fois saturé ou non saturé, ramifié ou non ramifié, substitué une ou plusieurs fois ou non substitué, un reste cycloalkyle en $C_3$ à Ce, saturé ou non saturé, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un radical alkylène en $C_1$ à $C_3$, chacun substitué une ou plusieurs fois ou non substitué ; SH, OH, F, Cl, I, Br, CN, $NO_2$, $NH_2$, $OR^{26}$;
$R^{26}$ étant choisi entre des restes alkyle en $C_1$ à $C_6$, saturés ou non saturés, ramifiés ou non ramifiés, substitués une ou plusieurs fois ou non substitués ;
avantageusement
$R^3$ est choisi entre H; un reste alkyle en $C_1$ à $C_6$, dans chaque cas saturé ou non saturé, ramifié ou non ramifié, substitué une ou plusieurs fois ou non substitué ; SH, OH, F, Cl, I, Br, CN, $NO_2$, $NH_2$, $OR^{26}$ ;
$R^{26}$ étant choisi entre des restes alkyle en $C_1$ à $C_6$, saturés ou non saturés, ramifiés ou non ramifiés, substitués une ou plusieurs fois ou non substitués ;
en particulier
$R^3$ représente H.

4. Dérivés substitués de 2-pyridine-cyclohexane-1,4-diamine suivant l'une des revendications 1 à 3, **caractérisés en ce que**
$R^4$ est choisi entre H, un groupe C (X) $R^7$, $C(X)NR^7R^8$, $C(X)OR^9$, C (X) $SR^9$ ou S ($O_2$) $R^9$ avec X = O ou S,
avantageusement
$R^4$ est choisi entre H, un groupe $C(X)R^7$, $C(X)NR^7R^8$ ou $C(X)OR^9$, avec X = 0,
en particulier
$R^4$ est choisi entre H ou un groupe $C(O)R^7$; $R^7$ étant avantageusement choisi entre
H; ou un reste alkyle en $C_1$ à $C_8$, saturé ou non saturé, ramifié ou non ramifié, substitué une ou plusieurs fois ou non substitué ;
avantageusement
H ; ou un reste alkyle en $C_1$ à $C_3$ saturé, non substitué, ramifié ou non ramifié ;
en particulier $CH_3$.

**5.** Dérivés substitués de 2-pyridine-cyclohexane-1,4-diamine suivant l'une des revendications 1 à 3, **caractérisés en ce que**
$R^4$ et $R^5$ forment ensemble un hétérocycle ayant entre 3 et 8 atomes dans le noyau, saturé ou non saturé ; substitué une ou plusieurs fois ou non substitué, avantageusement avec entre 5 et 7 atomes dans le noyau, dont le noyau contient, à côté de l'atome N obligatoire, 0 ou 1 autre hétéroatome choisi entre N, S ou O ;
l'hétérocycle formé conjointement par $R^4$ et $R^5$ pouvant éventuellement être condensé avec d'autres noyaux, avantageusement avec des noyaux aromatiques et/ou hétéroaromatiques, lesquels peuvent être condensés avec d'autres noyaux aromatiques et/ou hétéroaromatiques,
l'hétérocycle formé conjointement par $R^4$ et $R^5$ étant en particulier condensé avec un ou deux autres noyaux, l'hétérocycle formé conjointement par $R^4$ et $R^5$ étant avantageusement condensé avec deux autres noyaux de telle manière que $R^4$ et $R^5$ forment ensemble

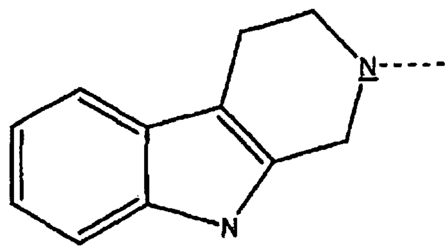
.

**6.** Dérivés substitués de 2-pyridine-cyclohexane-1,4-diamine suivant l'une des revendications 1 à 3, **caractérisés en ce que**
$R^4$ est choisi entre H ou un reste alkyle en $C_1$ à $C_8$, saturé ou non saturé, ramifié ou non ramifié, substitué une ou plusieurs fois ou non substitué, avantageusement
H ou un reste alkyle en $C_1$ à $C_6$, saturé ou non saturé, ramifié ou non ramifié, substitué une ou plusieurs fois ou non substitué,
en particulier
H ou un reste alkyle en $C_1$ à $C_3$, saturé, non ramifié et non substitué.

**7.** Dérivés substitués de 2-pyridine-cyclohexane-1,4-diamine suivant l'une des revendications 1 à 6,
**caractérisés en ce que**
$R^5$ est choisi entre un reste cycloalkyle en $C_3$ à $C_8$, aryle ou hétéroaryle, chacun non substitué ou substitué une ou plusieurs fois ;
avantageusement
$R^5$ est choisi entre un reste cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxannyle, benzodioxolanyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolanyle, adamantyle, pyrimidinyle, quinolinyle, isoquinolinyle, phtalazinyle ou quinazolinyle, chacun non substitué ou substitué une ou plusieurs fois;
en particulier
$R^5$ est choisi entre un reste cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, chacun non substitué ou substitué une ou plusieurs fois.

**8.** Dérivés substitués de 2-pyridine-cyclohexane-1,4-diamine suivant l'une des revendications 1 à 6, **caractérisés en ce que**
$R^5$ est choisi entre $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,
avec Y = O, S ou $H_2$,
avantageusement $R^5$ est choisi entre $-CHR^{11}R^{12}$, $-CHR^{11}-CH_2R^{12}$, $-CHR^{11}-CH_2-CH_2R^{12}$, $-C(Y)R^{12}$, $-C(Y)-CH_2R^{12}$ ou $-C(Y)-CH_2-CH_2R^{12}$,
avec Y = 0 ou S,
en particulier

$R^5$ est choisi entre $-CHR^{11}R^{12}$, $-CHR^{11}-CH_2R^{12}$, $-CHR^{11}-CH_2-CH_2R^{12}$, $-C(Y)R^{12}$ ou $-C(Y)-CH_2R^{12}$, avec Y = O.

**9.** Dérivés substitués de 2-pyridine-cyclohexane-1,4-diamine suivant la revendication 8, **caractérisé en ce que** $R^{11}$ est choisi entre
H, un reste alkyle en $C_1$ à $C_4$, saturé ou non saturé, ramifié ou non ramifié, substitué une ou plusieurs fois ou non substitué ; ou un groupe C(O)O-(alkyle en $C_1$ à $C_4$), saturé ou non saturé, ramifié ou non ramifié, substitué une ou plusieurs fois ou non substitué ;
avantageusement
H, un reste alkyle en $C_1$ à $C_4$, saturé ou non saturé, ramifié ou non ramifié, substitué une ou plusieurs fois ou non substitué ; ou un groupe C(O)O-(alkyle en $C_1$ ou $C_2$), saturé, non ramifié, substitué une ou plusieurs fois ou non substitué ;
en particulier
H, $CH_3$, $C_2H_5$ et $C(O)O-CH_3$.

**10.** Dérivés substitués de 2-pyridine-cyclohexane-1,4-diamine suivant la revendication 8, **caractérisés en ce que** $R^{12}$ est choisi entre un reste cycloalkyle en $C_3$ à $C_8$, aryle ou hétéroaryle, chacun non substitué ou substitué une ou plusieurs fois ;
avantageusement
$R^{12}$ est choisi entre un reste cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolanyle, adamantyle, pyrimidinyle, quinolinyle, isoquinolinyle, phtalazinyle ou quinazolinyle, chacun non substitué ou substitué une ou plusieurs fois ;
en particulier
$R^{12}$ est choisi entre un reste cyclopentyle, cyclohexyle, cycloheptyle, cyclo-octyle, anthracényle, indolyle, naphtyle, benzofurannyle, benzothiophényle, indanyle, benzodioxannyle, benzodioxolannyle, acénaphtyle, carbazolyle, phényle, thiophényle, furyle, pyridyle, pyrrolyle, pyrazinyle ou pyrimidinyle, chacun non substitué ou substitué une ou plusieurs fois.

**11.** Dérivés substitués de 2-pyridine-cyclohexane-1,4-diamine suivant l'une des revendications 1 à 10, **caractérisés en ce qu'**ils sont choisis dans le groupe suivant :

- dichlorhydrate de N-(4-diméthylamino-4-pyridine-2-yl-cyclohexyl)-N-[2-(1H-indole-3-yl)-éthyl]-acétamide, diastéréo-isomère le moins polaire
- trichlorhydrate de N'-[2-(1H-indole-3-yl)-éthyl]-N,N-diméthyl-1-pyridine-2-yl-cyclohexane-1,4-diamine, diastéréo-isomère le moins polaire
- trichlorhydrate de N'-[2-(1H-indole-3-yl)-éthyl]-N,N-diméthyl-1-pyridine-2-yl-cyclohexane-1,4-diamine, diastéréo-isomère le plus polaire
- trichlorhydrate d'ester méthylique d'acide (*S*)-2-(4-diméthylamino-4-pyridine-2-yl-cyclohexylamino)-3-(1H-indole-3-yl)-propionique, diastéréo-isomère le moins polaire
- trichlorhydrate d'ester méthylique d'acide (*S*)-2-(4-diméthylamino-4-pyridine-2-yl-cyclohexylamino)-3-(1H-indole-3-yl)-propionique, diastéréo-isomère le plus polaire
- dichlorhydrate d'acide (*S*)-2-(4-diméthylamino-4-pyridine-2-yl-cyclohexylamino)-3-(1H-indole-3-yl)-propionique, diastéréo-isomère le moins polaire, éventuellement aussi sous forme de leurs racémates, des stéréo-isomères mentionnés ou d'autres stéréo-isomères purs, en particulier d'énantiomères ou de diastéréo-isomères, ou sous forme de mélanges des stéréo-isomères, en particulier des énantiomères ou des diastéréo-isomères, dans un rapport de mélange quelconque ; éventuellement aussi sous forme des acides ou des bases

ou sous forme d'autres sels, en particulier de sels physiologiquement acceptables ou de sels d'acides ou de cations physiologiquement acceptables ; ou sous forme de leurs produits de solvatation, en particulier des hydrates.

**12.** Médicament contenant au moins un dérivé substitué de 2-pyridine-cyclohexane-1,4-diamine suivant l'une des revendications 1 à 11, le cas échéant sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de ses acides ou de ses bases ou sous forme de ses sels physiologiquement acceptables ou de sels

d'acides ou de cations physiologiquement acceptables ; ou sous forme de ses produits de solvatation, en particulier des hydrates, contenant aussi le cas échéant des additifs et/ou des substances auxiliaires convenables et/ou le cas échéant d'autres substances actives.

**13.** Médicament suivant la revendication 12, **caractérisé en ce qu'**il contient, à côté d'au moins un dérivé substitué de 2-pyridine-cyclohexane-1,4-diamine, un opiacé, avantageusement un opiacé puissant, en particulier la morphine, ou un anesthésique, avantageusement l'hexobarbital ou l'halothane.

**14.** Utilisation d'un dérivé substitué de 2-pyridine-cyclohexane-1,4-diamine suivant l'une des revendications 1 à 11, éventuellement sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de ses acides ou de ses bases ou sous forme de ses sels physiologiquement acceptables ou de sels d'acides ou de cations physiologiquement acceptables ; ou sous forme de ses produits de solvatation, en particulier des hydrates ; pour la préparation d'un médicament destiné au traitement de la douleur, en particulier d'une douleur neuropathique ou chronique aiguë.

**15.** Utilisation d'un dérivé substitué de 2-pyridine-cyclohexane,-1,4-diamine suivant l'une des revendications 1 à 11, le cas échéant sous forme de son racémate, des stéréo-isomères purs, en particulier des énantiomères ou des diastéréo-isomères, ou sous forme de mélanges des stéréo-isomères, en particulier des énantiomères ou des diastéréo-isomères, dans un rapport de mélange quelconque ; sous la forme représentée ou sous forme de ses acides ou de ses bases ou sous forme de ses sels physiologiquement acceptables ou de sels d'acides ou de cations physiologiquement acceptables ; ou sous forme de ses produits de solvatation, en particulier des hydrates ; pour la préparation d'un médicament destiné au traitement d'états d'anxiété, de stress et de syndromes liés au stress, de dépressions, de l'épilepsie, de la maladie d'Alzheimer, de la démence sénile, de troubles cognitifs généraux, de difficultés d'apprentissage et de mémoire (comme nootrope), de phénomènes de privation, d'usage abusif et/ou de dépendance à l'égard de l'alcool et/ou des drogues et/ou des médicaments, de dysfonctionnements sexuels, de maladies cardio-vasculaires, d'hypotension, d'hypertension, d'acouphènes, de prurit, de migraine, de difficulté auditive, de défaut de motilité de l'intestin, de troubles de l'ingestion de nourriture, de l'anorexie, de l'adiposité, de troubles locomoteurs, de diarrhée, de cachexie, d'incontinence d'urine, et comme relaxant musculaire, anticonvulsif ou anesthésique, ou en vue de la coadministration dans le traitement avec un analgésique opiacé ou avec un anesthésique, pour la diurèse ou l'anti-natriurèse et/ou pour l'anxiolyse.

**16.** Procédé de production d'un dérivé substitué de 2-pyridine-cyclohexane-1,4-diamine suivant l'une des revendications 1 à 11 avec $R^3$ = H, comprenant les étapes suivantes :

a. une cyclohexane-1,4-dione protégée avec les groupes $S^1$ et $S^2$ selon la formule II est amenée à réagir en présence d'un composé de formule $HNR^{01}R^{02}$ avec un cyanure, avantageusement le cyanure de potassium, pour former un dérivé de 1-amino-4-oxo-cyclohexanecarbonitrile substitué sur l'azote et protégé, de formule III ;

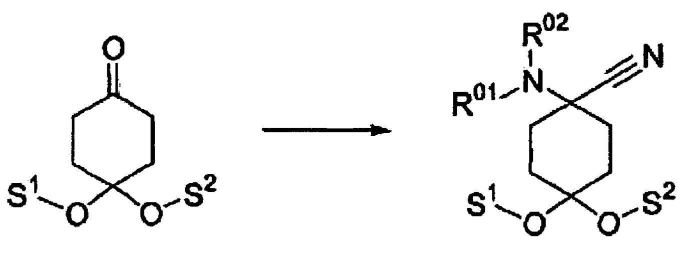

II III

le cas échéant, on procède ensuite dans un ordre quelconque et de façon éventuellement répétée à une acylation, une alkylation ou une sulfonation et/ou dans le cas de composés dans lesquels $R^{01}$ et/ou $R^{02}$ et/ou $R^{06}$ représentent un atome d'hydrogène protégé avec un groupe protecteur, on élimine au moins une fois un groupe protecteur et on procède éventuellement à une acylation, une alkylation ou une sulfonation et/ou dans le cas d'un composé dans lequel $R^{01}$ et/ou $R^{02}$ et/ou $R^{06}$ représentent H, on introduit au moins une fois un

groupe protecteur et on procède éventuellement à une acylation, une alkylation ou une sulfonation,

b. l'aminonitrile selon la formule III est mis en présence de cyclopentadiényl-cyclo-octa-1,5-diène-cobalt(I) [cpCo (cod)] et irradié sous acétylène, ce qui produit un composé de formule IVa ;

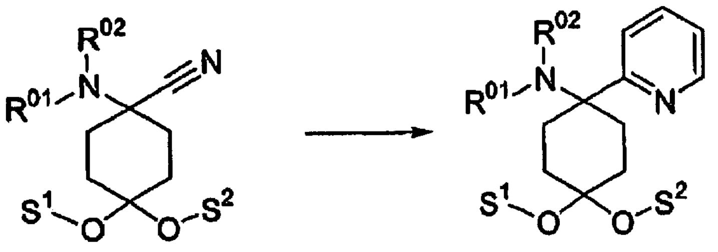

III IVa

on procède ensuite le cas échéant dans un ordre quelconque et de façon éventuellement répétée à une acylation, une alkylation ou une sulfonation et/ou dans le cas de composés dans lesquels $R^{01}$ et/ou $R^{02}$ et/ou $R^{06}$ représentent H protégé avec un groupe protecteur, on élimine au moins une fois un groupe protecteur et on procède éventuellement à une acylation, une alkylation ou une sulfonation et/ou dans le cas d'un composé dans lequel $R^{01}$ et/ou $R^{02}$ et/ou $R^{06}$ représentent H, on introduit au moins une fois un groupe protecteur et on procède éventuellement à une acylation, une alkylation ou une sulfonation,

c. on élimine les groupes protecteurs $S^1$ et $S^2$ du composé selon la formule IVa, ce qui donne un dérivé substitué en position 4 de 4-aminocyclohexanone de formule IV ;

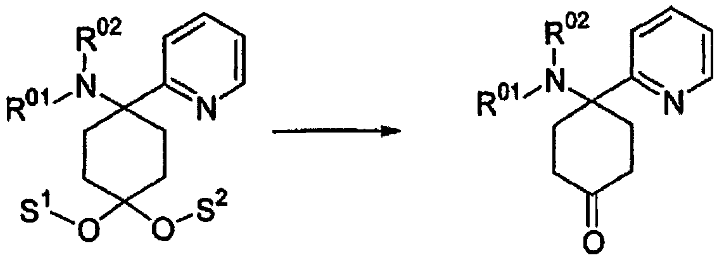

IVa IV

le cas échéant, on procède ensuite dans un ordre quelconque et de façon éventuellement répétée à une acylation, une alkylation ou une sulfonation et/ou dans le cas de composés dans lesquels $R^{01}$ et/ou $R^{02}$ et/ou $R^{06}$ représentent H protégé par un groupe protecteur, on élimine au moins une fois un groupe protecteur et on procède éventuellement à une acylation, une alkylation ou une sulfonation et/ou dans le cas d'un composé dans lequel $R^{01}$ et/ou $R^{02}$ et/ou $R^{06}$ représentent H, on introduit au moins une fois un groupe protecteur et on procède éventuellement à une acylation, une alkylation ou une sulfonation,

d. le dérivé de 4-amino-cyclohexanone substitué en position 4 selon la formule IVa est aminé par voie de réduction avec un composé de formule $HNR^{04}R^{05}$, ce qui produit un dérivé de 2-pyridine-cyclohexane-1,4-diamine de formule V ;

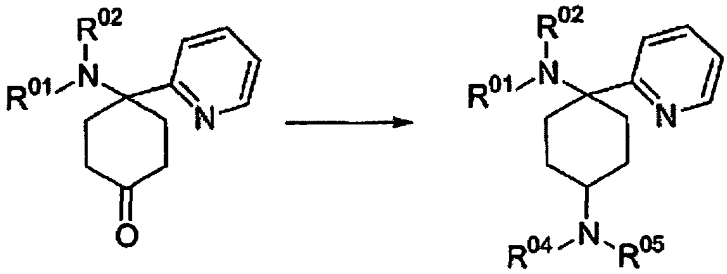

IV V

le cas échéant, on procède ensuite dans un ordre quelconque et de façon éventuellement répétée à une acylation, une alkylation ou une sulfonation et/ou dans le cas de composés dans lesquels $R^{01}$ et/ou $R^{02}$ et/ou $R^{04}$ et/ou $R^{05}$ et/ou $R^{06}$ représentent H protégé par un groupe protecteur, on élimine au moins une fois un groupe protecteur et on effectue éventuellement une acylation, une alkylation ou une sulfonation et/ou dans le cas d'un composé dans lequel $R^{01}$ et/ou $R^{02}$ et/ou $R^{04}$ et/ou $R^{05}$ et/ou $R^{06}$ représentent H, on introduit au moins une fois un groupe protecteur et on effectue éventuellement une acylation, une alkylation

ou une sulfonation, jusqu'à ce que soit produit un composé de formule I,
dans laquelle $R^1$, $R^2$, $R^4$ et $R^5$ ont la définition indiquée dans la revendication 1
et
$R^{01}$ et $R^{02}$ sont choisis, indépendamment l'un de l'autre, entre H ; H muni d'un groupe protecteur ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un radical alkylène en $C_1$ à $C_3$, chacun substitué une ou plusieurs fois ou non substitué ;
ou bien les restes $R^{01}$ et $R^{02}$ forment ensemble un noyau et représentent $CH_2CH_2OCH_2CH_2$, $CH_2CH_2NR^{06}CH_2CH_2$ ou $(CH_2)_{3-6}$,
$R^{06}$ étant choisi entre H; H muni d'un groupe protecteur ; un reste alkyle en $C_1$ à $C_8$ ou cycloalkyle en $C_3$ à $C_8$, chacun saturé ou non saturé, ramifié ou non ramifié, substitué une ou plusieurs fois ou non substitué ; un reste aryle ou hétéroaryle, chacun substitué une ou plusieurs fois ou non substitué ; ou un reste aryle, cycloalkyle en $C_3$ à $C_8$ ou hétéroaryle lié par l'intermédiaire d'un radical alkylène en $C_1$ à $C_3$, chacun substitué une ou plusieurs fois ou non substitué ;
$R^{04}$ est choisi entre H, H muni d'un groupe protecteur ; un reste alkyle en $C_1$ à $C_8$, saturé ou non saturé, ramifié ou non ramifié, substitué une ou plusieurs fois ou non substitué ;
$R^{05}$ est choisi entre H, H muni d'un groupe protecteur ; un reste cycloalkyle en $C_3$ à $C_8$, aryle ou hétéroaryle, chacun non substitué ou substitué une ou plusieurs fois ; $-CHR^{11}R^{12}$, $-CHR^{11}-CH_2R^{12}$, $-CHR^{11}-CH_2-CH_2R^{12}$, $-CHR^{11}-CH_2-CH_2-CH_2R^{12}$, $-C(Y)R^{12}$, $-C(Y)-CH_2R^{12}$, $-C(Y)-CH_2-CH_2R^{12}$ ou $-C(Y)-CH_2-CH_2-CH_2R^{12}$,
avec $Y = H_2$,
$R^{11}$ étant choisi entre
H, un reste alkyle en $C_1$ à $C_7$, saturé ou non saturé, ramifié ou non ramifié, substitué une ou plusieurs fois ou non substitué ;
et $R^{12}$ étant choisi entre
H ; un reste cycloalkyle en $C_3$ à $C_8$, aryle ou hétéroaryle, chacun non substitué ou substitué une ou plusieurs fois,
ou bien $R^{04}$ et $R^{05}$ forment ensemble un hétérocycle avec entre 3 et 8 atomes dans le noyau, saturé ou non saturé ; substitué une ou plusieurs fois ou non substitué,
et $S^1$ et $S^2$ sont choisis indépendamment l'un de l'autre entre des groupes protecteurs ou forment ensemble un groupe protecteur, avantageusement monoacétal.

**17.** Procédé de production d'un dérivé substitué de 2-pyridine-cyclohexane-1,4-diamine suivant l'une des revendications 1 à 11 avec $R^3$ = H, comprenant les étapes suivantes :

a. une cyclohexane-1,4-dione protégée avec les groupes $S^1$ et $S^2$ répondant à la formule II est aminée par voie de réduction avec un composé de formule $HNR^{04}R^{05}$ de manière à produire un dérivé de 4-aminocyclohexanone de formule VI ;

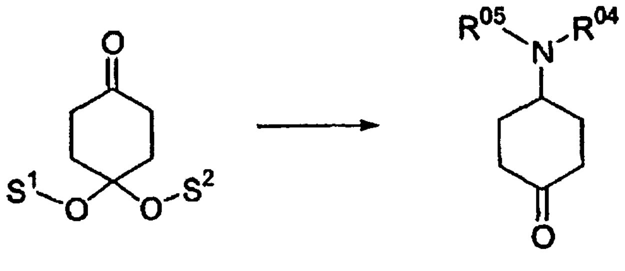

II VI

on effectue ensuite le cas échéant, dans un ordre quelconque et éventuellement de façon répétée, une acylation, une alkylation ou une sulfonation et/ou dans le cas de composés dans lesquels $R^{04}$ et/ou $R^{05}$ représentent H protégé avec un groupe protecteur, on élimine au moins une fois un groupe protecteur et on effectue éventuellement une acylation, une alkylation ou une sulfonation et/ou dans le cas d'un composé dans lequel $R^{04}$ et/ou $R^{05}$ représentent H, on introduit au moins une fois un groupe protecteur et on effectue éventuellement une acylation, une alkylation ou une sulfonation,

b. le dérivé de 4-aminocyclohexanone répondant à la formule VI est amené à réagir en présence d'un composé de formule $HNR^{01}R^{02}$ avec un cyanure, avantageusement le cyanure de potassium, pour former un dérivé nitrilique de cyclohexanone de formule VII,

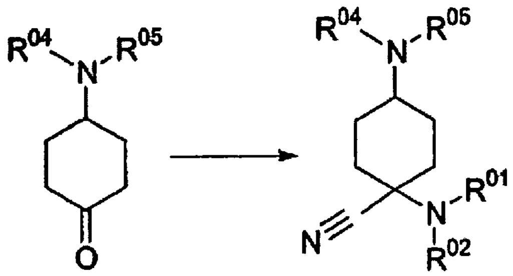

VI VII

on effectue ensuite le cas échéant, dans un ordre quelconque et éventuellement de façon répétée, une acylation, une alkylation ou une sulfonation et/ou dans le cas de composés dans lesquels $R^{01}$ et/ou $R^{02}$ et/ou $R^{04}$ et/ou $R^{05}$ et/ou $R^{06}$ représentent H protégé avec un groupe protecteur, on élimine au moins une fois un groupe protecteur et on effectue éventuellement une acylation, une alkylation ou une sulfonation et/ou dans le cas d'un composé dans lequel $R^{01}$ et/ou $R^{02}$ et/ou $R^{04}$ et/ou $R^{05}$ et/ou $R^{06}$ représentent H, on introduit au moins une fois un groupe protecteur et on effectue le cas échéant une acylation, une alkylation ou une sulfonation,

c. le dérivé nitrilique de cyclohexanone de formule VII est mis en présence de cyclopentadiényl-cyclo-octa-1,5-diène-cobalt(I) [cpCo(cod)] et irradié sous acétylène, ce qui produit un dérivé de 2-pyridine-cyclohexane-1,4-diamine de formule V,

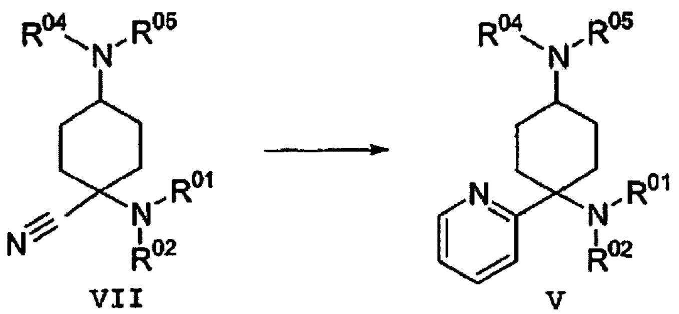

VII V

on effectue ensuite le cas échéant, dans un ordre quelconque et éventuellement de façon répétée, une acylation, une alkylation ou une sulfonation et/ou dans le cas de composés dans lesquels $R^{01}$ et/ou $R^{02}$ et/ou $R^{04}$ et/ou $R^{05}$ et/ou $R^{06}$ représentent H protégé avec un groupe protecteur, on élimine au moins une fois un groupe protecteur et on effectue éventuellement une acylation, une alkylation ou une sulfonation et/ou dans le cas d'un composé dans lequel $R^{01}$ et/ou $R^{02}$ et/ou $R^{04}$ et/ou $R^{05}$ et/ou $R^{06}$ représentent H, on introduit au moins une fois un groupe protecteur et on effectue le cas échéant une acylation, une alkylation ou une sulfonation, jusqu'à ce que soit produit un composé de formule I, $R^1$, $R^2$, $R^4$ et $R^5$ ayant la définition indiquée dans la revendication 1, et les restes $R^{01}$, $R^{02}$, $R^{06}$, $R^{04}$, $R^{05}$, $R^{11}$, $R^{12}$, $S^1$ et $S^2$ ayant la définition indiquée dans la revendication 16.

**18.** Procédé de production d'un dérivé substitué de 2-pyridine-cyclohexane-1,4-diamine suivant l'une des revendications 16 ou 17, **caractérisé en ce que** les groupes protecteurs sur H pour $R^{01}$, $R^{02}$, $R^{04}$, $R^{05}$ et/ou $R^{06}$ sont choisis entre des groupes alkyle, benzyle ou des groupes carbamate, par exemple FMOC, Z ou Boc.

**19.** Procédé de production d'un dérivé substitué de 2-pyridine-cyclohexane-1,4-diamine suivant la revendication 16, **caractérisé en ce que** l'amination par voie de réduction dans l'étape d a lieu en présence de formiate d'ammonium, d'acétate d'ammonium ou de $NaCNBH_3$.

**20.** Procédé de production d'un dérivé substitué de 2-pyridine-cyclohexane-1,4-diamine suivant l'une des revendications 1 à 11, avec $R^3$ = H, comprenant les étapes suivantes:

a. une cyclohexane-1,4-dione protégée avec les groupes $S^1$ et $S^2$ selon la formule II est amenée à réagir en présence d' un composé de formule $HNR^{01}R^{02}$ avec un cyanure, avantageusement le cyanure de potassium, pour former un dérivé N-substitué protégé de 1-amino-4-oxo-cyclohexanecarbonitrile selon la formule III ;

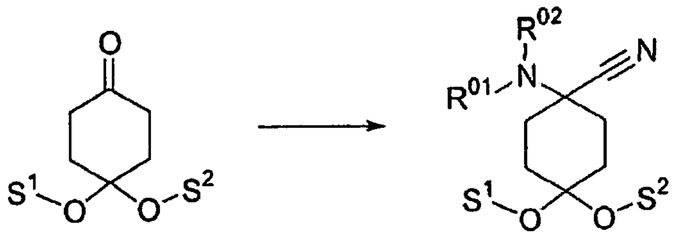

II III

on conduit ensuite le cas échéant, dans un ordre quelconque et de façon éventuellement répétée, une acylation, une alkylation ou une sulfonation et/ou, dans le cas de composés dans lesquels $R^{01}$ et/ou $R^{02}$ et/ou $R^{06}$ représentent H protégé avec un groupe protecteur, on élimine au moins une fois un groupe protecteur et on effectue le cas échéant une acylation, une alkylation ou une sulfonation et/ou dans le cas d'un composé dans lequel $R^{01}$ et/ou $R^{02}$ et/ou $R^{06}$ représentent H, on introduit au moins une fois un groupe protecteur et on effectue éventuellement une acylation, une alkylation ou une sulfonation,

b. l'aminonitrile selon la formule III est mis en présence de cyclopentadiényl-cyclo-octa-1,5-diéne-cobalt(I) [cpCo (cod)] et irradié sous acétylène, ce qui produit un composé de formule IVa ;

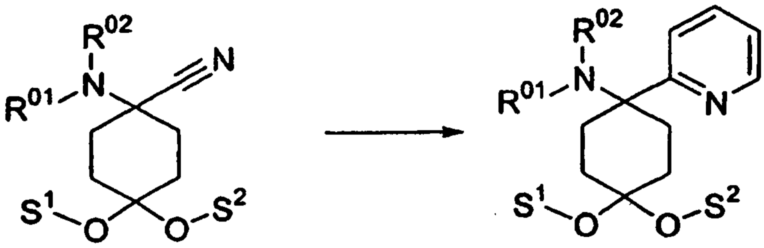

III IVa

le cas échéant, on conduit ensuite, dans un ordre quelconque et de façon éventuellement répétée, une acylation, une alkylation ou une sulfonation et/ou dans le cas de composés dans lesquels $R^{01}$ et/ou $R^{02}$ et/ou $R^{06}$ représentent H protégé avec un groupe protecteur, on élimine au moins une fois un groupe protecteur et on effectue le cas échéant une acylation, une alkylation ou une sulfonation et/ou dans le cas d'un composé dans lequel $R^{01}$ et/ou $R^{02}$ et/ou $R^{06}$ représentent H, on introduit au moins une fois un groupe protecteur et on effectue éventuellement une acylation, une alkylation ou une sulfonation,

c. on élimine sur le composé de formule IVa les groupes protecteurs $S^1$ et $S^2$ de manière à produire un dérivé 4-substitué de 4-aminocyclohexanone de formule IV ;

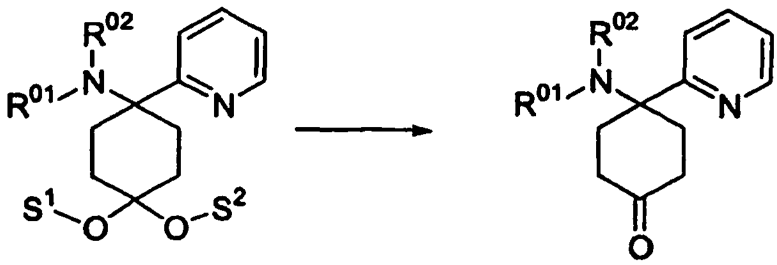

IVa IV

le cas échéant, on effectue ensuite dans un ordre quelconque et éventuellement de façon répétée une acylation, une alkylation ou une sulfonation et/ou dans le cas de composés dans lesquels $R^{01}$ et/ou $R^{02}$ et/ou $R^{06}$ représentent H protégé avec un groupe protecteur, on élimine au moins une fois un groupe protecteur et on conduit éventuellement une acylation, une alkylation ou une sulfonation et/ou dans le cas d'un composé dans lequel $R^{01}$ et/ou $R^{02}$ et/ou $R^{06}$ représentent H, on introduit au moins une fois un groupe protecteur et on conduit éventuellement une acylation, une alkylation ou une sulfonation,

d. le composé IV réagit avec l'hydroxylamine et est réduit après formation de l'oxime,

on conduit ensuite le cas échéant, dans un ordre quelconque et de façon éventuellement répétée, une acylation, une alkylation ou une sulfonation et/ou dans le cas de composés dans lesquels $R^{01}$ et/ou $R^{02}$ et/ou $R^{04}$ et/ou $R^{05}$ et/ou $R^{06}$ représentent H protégé par un groupe protecteur, on élimine au moins une fois un groupe protecteur et on conduit éventuellement une acylation, une alkylation ou une sulfonation et/ou dans le cas d'un composé dans lequel $R^{01}$ et/ou $R^{02}$ et/ou $R^{04}$ et/ou $R^{05}$ et/ou $R^{06}$ représentent H, on introduit au moins une fois un groupe protecteur et on conduit éventuellement une acylation, une alkylation

ou une sulfonation, jusqu'à ce soit produit un composé de formule I,
dans laquelle $R^1$, $R^2$, $R^4$ et $R^5$ ont la définition indiquée dans la revendication 1
et les restes $R^{01}$, $R^{02}$, $R^{06}$, $R^{04}$, $R^{05}$, $R^{11}$, $R^{12}$, $S^1$ et $S^2$ ont la définition indiquée dans la revendication 16.

**21.** Procédé de production d'un dérivé substitué de 2-pyridine-cyclohexane-1,4-diamine suivant la revendication 16, **caractérisé en ce que** l'irradiation dans l'étape b a une durée de 5 à 7 heures et/ou a lieu à la température ambiante et/ou en atmosphère saturée d'acétylène et/ou sous gaz protecteur.

**22.** Procédé de production d'un dérivé substitué de 2-pyridine-cyclohexane-1,4-diamine suivant la revendication 17, **caractérisé en ce que** l'irradiation dans l'étape c a une durée de 5 à 7 heures et/ou est conduite à la température ambiante et/ou en atmosphère saturée d'acétylène et/ou sous gaz protecteur.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **MEUNIER et al.** *Nature,* 1995, vol. 377, 532-535
- **MOLLEREAU et al.** *FEBS Letters,* 1994, vol. 341, 33-38
- **DARLAND et al.** *Trends in Neurosciences,* 1998, vol. 21, 215-221
- **ARDATI et al.** *Mol. Pharmacol.,* vol. 51, 816-824
- **MATTHES et al.** *Mol. Pharmacol.,* 1996, vol. 50, 447-450
- **VAUGHAN et al.** *Br. J. Pharmacol.,* 1996, vol. 117, 1609-1611
- **CONNER et al.** *Br. J. Pharmacol.,* 1996, vol. 118, 205-207
- **KNOFLACH et al.** *J. Neuroscience,* 1996, vol. 16, 6657-6664
- **REINSCHEID et al.** *Science,* 1995, vol. 270, 792-794
- **HARA et al.** *Br. J. Pharmacol.,* 1997, vol. 121, 401-408
- **MOGIL et al.** *Neurosci. Letters,* 1996, vol. 214, 131-134
- *Neuroscience,* 1996, vol. 75, 333-337
- **JENCK et al.** *Proc. Natl. Acad. Sci. USA,* 1997, vol. 94, 14854-14858
- **SHU et al.** *Neuropeptides,* 1998, vol. 32, 567-571
- **FABER et al.** *Br. J. Pharmacol.,* 1996, vol. 119, 189-190
- **KING et al.** *Neurosci. Lett.,* 1997, vol. 223, 113-116
- **XU et al.** *NeuroReport,* 1996, vol. 7, 2092-2094
- **YAMAMOTO et al.** *Neuroscience,* 1997, vol. 81, 249-254
- **ABDULLA ; SMITH.** *J. Neurosci.,* 1998, vol. 18, 9685-9694
- **SANDIN et al.** *Eur. J. Neurosci.,* 1997, vol. 9, 194-197
- **MANABE et al.** *Nature,* 1997, vol. 394, 577-581
- **NISHI et al.** *EMBO J.,* 1997, vol. 16, 1858-1864
- **POMONIS et al.** *NeuroReport,* 1996, vol. 8, 369-371
- **GUMUSEL et al.** *Life Sci.,* 1997, vol. 60, 141-145
- **CAMPION ; KADOWITZ.** *Biochem. Biophys. Res. Comm.,* 1997, vol. 234, 309-312
- **GUTIÉRREZ et al.** *Society for Neuroscience,* 07. November 1998, vol. 24
- **KAPISTA et al.** *Life Sciences,* 1997, vol. 60, 15-21
- **CALO et al.** *Br.J. Pharmacol.,* 2000, vol. 129, 1261-1283
- **ARDATI et al.** *Mol. Pharmacol.,* 1997, vol. 51, 816-824